(19) Europäisches Patentamt
European Patent Office
Office européen des brevets

(11) **EP 4 103 120 B1**

(12) **EUROPEAN PATENT SPECIFICATION**

(45) Date of publication and mention
of the grant of the patent:
**01.10.2025 Bulletin 2025/40**

(21) Application number: **21710692.1**

(22) Date of filing: **11.02.2021**

(51) International Patent Classification (IPC):
*A61F 13/15* (2006.01)   *A61F 13/505* (2006.01)
*A61F 13/62* (2006.01)   *A61F 13/74* (2006.01)

(52) Cooperative Patent Classification (CPC):
**A61F 13/15268; A61F 13/505; A61F 13/622;
A61F 13/74**

(86) International application number:
**PCT/US2021/017561**

(87) International publication number:
**WO 2021/163258 (19.08.2021 Gazette 2021/33)**

(54) **ABSORBENT ARTICLE WITH FASTENING SYSTEM**

SAUGFÄHIGER ARTIKEL MIT BEFESTIGUNGSVORRICHTUNGSSYSTEM

ARTICLE ABSORBANT AVEC SYSTÈME DE FIXATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.02.2020 US 202062975836 P
21.09.2020 US 202063080929 P**

(43) Date of publication of application:
**21.12.2022 Bulletin 2022/51**

(73) Proprietor: **The Procter & Gamble Company
Cincinnati, OH 45202 (US)**

(72) Inventors:
• **ROE, Donald Carroll
Cincinnati, Ohio 45202 (US)**

• **SCHOENBORN, Udo Friedel
65824 Schwalbach an Taunus (DE)**
• **VANDERKLAAUW, Nicholette Johanna Birgitta
Maria
Cincinnati, Ohio 45202 (US)**

(74) Representative: **P&G Patent Germany
Procter & Gamble Service GmbH
Sulzbacher Straße 40
65824 Schwalbach am Taunus (DE)**

(56) References cited:
US-A1- 2012 022 485    US-A1- 2012 022 491
US-A1- 2013 006 209    US-A1- 2013 324 959
US-A1- 2014 000 003    US-A1- 2014 005 621
US-A1- 2014 257 226    US-A1- 2018 228 664

**Description**

FIELD OF THE INVENTION

The present invention relates generally to the field of wearable absorbent articles having absorbent inserts and reusable outer covers, and more particularly, to fastening systems for such absorbent articles.

BACKGROUND OF THE INVENTION

It has long been known that absorbent articles (*e.g.*, diapers, adult incontinence articles, feminine hygiene pads) offer the benefit of receiving and containing urine and/or other bodily exudates (*e.g.*, feces, menses, mixture of feces and urine, mixture of menses and urine, etc.). Absorbent articles may be made of reusable materials, which offers the advantage of utilizing raw materials from renewable and recyclable resources and less waste. However, reusable absorbent articles present difficulties relating to sanitation needs, in handling, laundering and effectively sanitizing soiled diapers for re-use. Further, reusable cloth diapers may be relatively unreliable and leak and may promote over-hydration of the wearer's skin causing diaper rash.

On the other hand, absorbent articles may be fully disposable, increasing consumer convenience by eliminating the need for laundering. Further, many current disposable absorbent articles have structures that make them relatively more effective at containing exudates and conveying and storing liquid exudates away from the wearer's skin. Some have features that enable them to "breathe", thereby reducing humidity inside the diaper, and some even include skin care compositions that are transferred to the skin when the diaper is worn. Although disposable conventional absorbent articles have many benefits, there is a continuous desire to reduce disposable material consumption and further improve consumer acceptance of single use products.

To address the foregoing concerns, it has been proposed to manufacture two-piece absorbent articles with a reusable outer cover and a detachable absorbent insert that may be reusable or disposable. In this way, the insert can be made with different materials to enhance performance and less energy may be consumed as the insert can be separately laundered. Further, where disposable, the inserts may be made with materials known to provide even more superior performance while minimizing the amount of waste as the whole article need not be disposed. Despite several designs of a two-piece absorbent article, the designs still present some disadvantages. In particular, articles may not provide sufficient attachment between the insert and outer cover to ensure a snug and comfortable fit. Further, known constructions utilize additional materials (e.g., adhesive) which increase manufacturing costs and may be less, or perceived to be less, environmentally friendly. Other constructions utilize buttons, slot/loops, or other fastening systems which may reduce the amount of material used but often do not permit easy or suitable adjustments for the wearer, may not retain fit after loading of the article and/or create complexity in article manufacturing. In addition, known constructions are limited in their ability to provide different fastening properties (e.g., greater peel strength) in different areas of the fastening system.

Thus, there is an ongoing need for a two-piece absorbent article that requires less material and can be made without undesirable complexity. Further, there is a need for a two-piece absorbent article that maintains desired fit during wear and after loading. There is also a need to provide targeted fastening zones in a cost efficient manner.

US2012022485A1 relates to a two-piece wearable absorbent article (such as a diaper) including an outer cover and an absorbent insert for use therewith.

US2013006209 relates to a two-piece wearer absorbent article having an outer cover and an absorbent insert.

SUMMARY OF THE INVENTION

The invention provides an absorbent article according to claim 1. Optional features are defined by the dependent claims. An absorbent article comprises an absorbent insert, an outer cover, and a fastening system. The fastening system comprises a fastening component and a receiving component operative engageable with the fastening component. The receiving component is disposed on a surface of one of the absorbent insert or the outer cover, the surface comprising a surface area. The fastening component is disposed on another of the absorbent insert or outer cover. The receiving component covers at least 50% of the surface area.

An absorbent article comprises an absorbent insert and an outer cover. The absorbent insert comprises a topsheet, a backsheet, and an absorbent core disposed between the topsheet and backsheet; a longitudinal axis and a lateral axis; and a first longitudinal edge and a second longitudinal edge, and a width in a lateral direction extending between the first and second longitudinal edges. The absorbent insert also comprises a fastening component disposed on a garment-facing surface and comprising integrally formed hooks; and an absorbent capacity of at least 120 g. The outer cover comprises a receiving component on a wearer-facing surface and operatively engageable with the fastening component.

An absorbent article comprises an absorbent insert, an outer cover and a fastening system. The absorbent insert comprises a topsheet, a backsheet, and an absorbent core disposed between the topsheet and backsheet. The fastening system comprises a fastening component and a receiving component, the fastening and receiving components being operatively engageable to join the absorbent insert to the outer cover or to enclose the absorbent insert within the outer cover, wherein the fastening component comprises a first macro pattern of fastening elements and a second macro pattern of fastening elements, wherein the first and second macro patterns differ by design element, surface area, opacity, color, average size of fastening elements within an array, average spacing of fastening elements within an array, aggregate shapes of arrays, directionality of fastening elements, orientation of arrays, whether fastening elements are discrete or integral or some combination, fastening element constituent materials, the number of fastening elements, the number and/or types of layers from which integral fastening elements are formed, spacing of arrays from one another, and combinations thereof.

BRIEF DESCRIPTION OF THE DRAWINGS

Fig. 1 is a perspective view of a wearable absorbent article as it might appear being worn by a wearer about the lower torso;
Fig. 2A is a plan view of an outer cover opened and laid flat, inner surface facing the viewer;
Fig. 2B is a plan view of an insert opened and laid flat, outer (garment-facing) surface facing the viewer;
Fig. 2C is a plan view of an outer cover opened and laid flat, outer surface facing the viewer;
Fig. 3 is a perspective view of a disposable absorbent insert shown apart from an outer cover, as it might appear in a free-standing, relaxed state;
Fig. 4 is a plan view of a disposable absorbent insert shown stretched out and laid flat, wearer-facing surfaces facing the viewer;
Fig. 5 is a cross sectional view of an example of an insert such as shown in Fig. 4, taken at line 5-5 in Fig. 4;
Figs. 6A-6B are schematic lateral cross sectional views of examples of inserts having a dual leg gasketing system, taken through a lateral axis of the insert example;
Figs. 7A-7C are depictions of various examples of macro patterns of fastening elements;
Figs. 8A-8C, 9A-9C, and 10A-10C depict front, side and top views of examples of profiles of hooks protruding from a substrate;
Fig. 11 is a plan view of a component comprising a fastening component disposed in a macro pattern;
Figs. 12A-12C are schematic side elevation views of exemplary hook configurations; and
Fig. 13 is a plan view of an insert opened and laid flat, inner surface facing the viewer.

DETAILED DESCRIPTION OF THE INVENTION

"Absorbent article" means a device that absorbs and contains body exudates and, more specifically, devices that are placed against or in proximity to the body of the wearer to absorb and contain the various exudates discharged from the body. Exemplary absorbent articles include diapers, training pants, pull-on pant-type diapers (i.e., a diaper having a pre-formed waist opening and leg openings such as illustrated in U.S. Patent No. 6,120,487), refastenable diapers or pant-type diapers, incontinence briefs and undergarments, diaper holders and liners, feminine hygiene garments such as panty liners, absorbent inserts, and the like.

"Absorbent insert" and "insert" mean a component of a wearable absorbent article that is adapted to contain and/or absorb urine, feces, menses or any combination thereof, and is adapted to be installable and removable as a modular unit, from an outer cover. Herein, an absorbent insert may also be referred to as an "absorbent assembly". The terms "absorbent insert," "insert" and "absorbent assembly" may be used interchangeably herein.

"Chassis" means a component of a wearable absorbent article that is adapted to be worn about the lower torso of a wearer, and is adapted to support an absorbent insert and hold the insert next to the wearer's body. Herein, a chassis may also be referred to as an "outer cover". The terms "outer cover" and "chassis" are interchangeable for purposes herein.

"Design element" as used herein means a shape or combination of shapes that visually create a distinct and discrete form, regardless of the size or orientation of the form. Design elements may comprise insignia. Design elements and/or combinations of design elements may comprise letters, words and/or graphics such as flowers, butterflies, hearts, character representations and the like. Design elements and/or combinations of design elements may comprise instructional indicia providing guidance or instruction to the caregiver relative to placement and/or fit of the article about the wearer.

"Insignia" as used herein means objects, character representations, words, colors, shapes or other indicia that can be used to distinguish, identify or represent the manufacturer, retailer, distributor and/or brand of a product, including but not limited to trademarks, logos, emblems, symbols, designs, figures, fonts, lettering, crests or similar identifying marks.

"Disposed" refers to an element being located in a particular place or position. A feature that is disposed on a surface or side of a component may be integral with said component or may be joined to said component.

"Disposable," when referring to an absorbent insert, means that the absorbent insert is not adapted or intended to be effectively sanitarily laundered in an ordinary household laundering process and ordinary household equipment, and thereby is ordinarily unsuitable for sanitary and effective reuse so as to provide as-new intended functions and performance, following soiling by exudates and removal from an outer cover. By way of non-limiting examples, effective laundering may be frustrated or prevented, causing the insert to be disposable, by inclusion of materials and/or construction: that do not retain their substantial as-new physical shape or structure through ordinary household laundering and drying so as to be effective as-new in reuse; that absorb aqueous liquids and cannot be sufficiently dried/dehydrated in ordinary household drying equipment and ordinary drying cycles so as to be effective as-new in reuse; that dissolve or substantially degrade in ordinary household laundering or drying, causing the insert to be substantially damaged or rendered useless; and/or that cannot be effectively cleaned of exudate material through ordinary laundering, so as to be sanitary and otherwise acceptable for re-use.

"Elastic" and "elastomeric" mean the ability of a material to stretch by at least 50% without rupture or breakage at a given load, and upon release of the load the elastic material or component exhibits at least 70% recovery (i.e., has less than 30% set) in one of the directions as per the Hysteresis Test described herein. Stretch, sometimes referred to as strain, percent strain, engineering strain, draw ratio, or elongation, along with recovery and set may each be determined according to the Hysteresis Test described in more detail below. Materials that are not elastic are referred as inelastic.

"Extensible" means the ability to stretch or elongate, without rupture or breakage, by at least 50% as per step 6(a) in the Hysteresis Test herein.

"Inboard," with respect to a first feature of an article and its position relative a second feature or location on the article, means that the first feature lies closer to a respective axis of the article than the second feature or location, along a horizontal x-y plane approximately occupied by the article when laid out flat, extended to the full longitudinal and lateral dimensions of its component web materials against any contraction induced by any included pre-strained elastomeric material, on a horizontal surface. Laterally inboard means the first feature is closer to the longitudinal axis, and longitudinally inboard means the first feature is closer to the lateral axis. Conversely, "outboard," with respect to a first feature of an article and its position relative a second feature or location on the article, means that the first feature lies farther from the respective axis of the article than the second feature or location.

"Integral" means configurations whereby an element is created from or created by an article component, or portions thereof, as opposed to being joined to the component. "Integrally formed" means an element is created from an underlying material or portion thereof, by for example molding, shaping and/or reconstituting the material.

"Joined" means configurations whereby an element is directly secured to another element by affixing the element directly to the other element, and configurations whereby an element is indirectly secured to another element by affixing the element to intermediate member(s) that in turn are affixed to the other element.

"Longitudinal" means a direction lengthwise in a component such that the longitudinal direction runs parallel to the maximum linear dimension in the x-y plane of the component. In an absorbent article as described herein, the longitudinal direction runs substantially perpendicular from a waist end edge to an opposing waist end edge when the absorbent article is in a flat out, uncontracted state, or from a waist end edge to the bottom of the crotch in a bifolded article.

"Lateral" refers to a direction generally perpendicular to the longitudinal direction. In the absorbent article described herein, the lateral direction runs substantially parallel from a side edge to an opposing side edge.

"Outer cover" means a component of a wearable absorbent article that is adapted to be worn about the lower torso of a wearer, and is adapted to support an absorbent insert and hold the insert next to the wearer's body. Herein, an outer cover may also be referred to as a "chassis". The terms "outer cover" and "chassis" are interchangeable for purposes herein.

"Reusable," when referring to a component means that the component is adapted to be used for its intended purpose after initial use without substantial destruction of any portions of the component necessary for as-new functionality. For example, a reusable outer cover means an outer cover that is adapted to permit removal of at least a first insert, and replacement thereof with at least a second insert, without substantial destruction of any portions of the outer cover that are necessary to provide the substantial as-new functionality of the outer cover, and without the necessity of any repair or reconstruction following such insert replacement.

"User" means a caregiver or other person who may apply an absorbent article to a wearer. Where the wearer is capable of donning the wearable absorbent article him/herself, the wearer is also a "user".

Overview

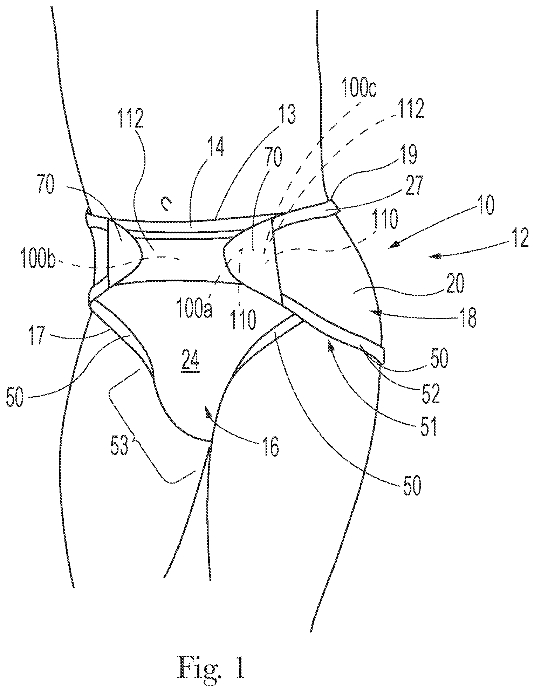

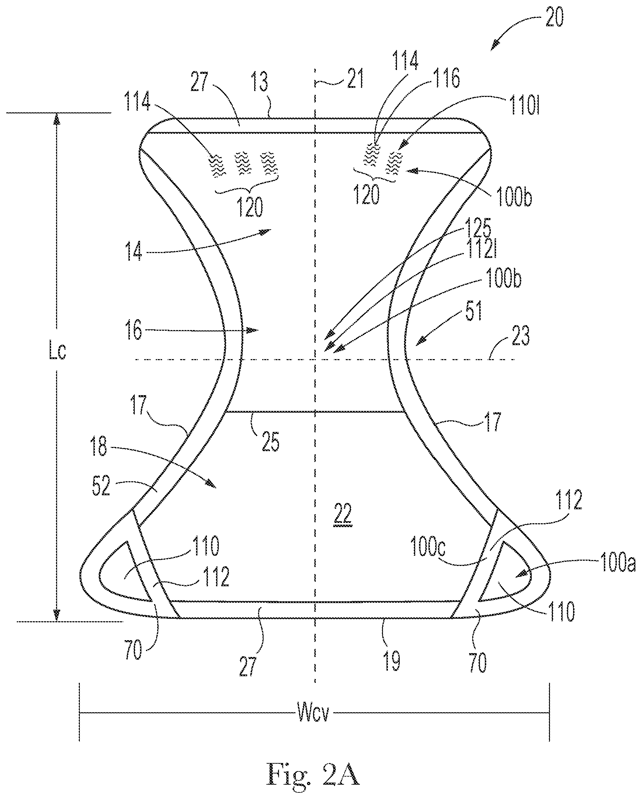

Fig. 1 is a perspective view an exemplary, nonlimiting embodiment of an absorbent article 10. The article is shown in the form of a two-piece absorbent article 12, having an outer cover 20 and an absorbent insert 30 shown in Figs. 2A-2B. It is to be understood that during manufacturing, the article may comprise several discrete pieces that are joined together. However, by two-piece absorbent article, it is meant that the article in its final form has two components that the user assembles together for wear. While shown as a two-piece absorbent article, it is also contemplated that the article may comprise more than two pieces, such as attachable ears or belts. Returning to Fig. 1, it can be seen that the absorbent article 10 may have a front region 14, a rear region 18 and a crotch region 16 disposed between the front and rear regions. The article may be placed on a wearer by wrapping the outer cover 20 between the wearer's legs and under the buttocks such that the crotch region 16 is between the wearer's legs. When the insert 30 has been installed into outer cover 20, the insert 30 will then be disposed within outer cover 20, next to the wearer. Nonlimiting examples of two-piece articles are disclosed in U.S. Pat. Nos. 8,998,870, 9,089,456, 8,435,223, 9,011,402, 8,808,263, 8,759,605 and 8,932,273 and 9,078,789.

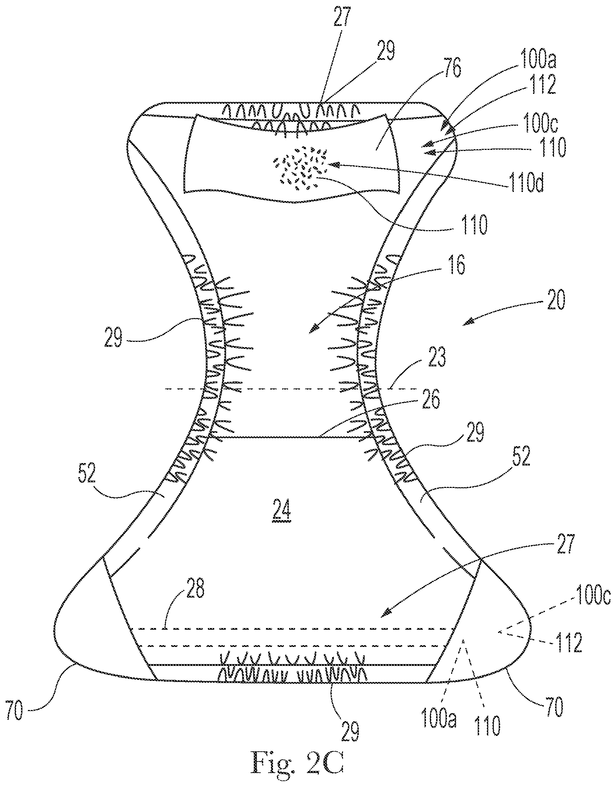

The absorbent article may comprise one or more fastening systems 100, which may be utilized, for example, to join the front and rear regions, to join the absorbent insert to the outer cover, and/or to join the outer cover to itself in an envelope fashion about the absorbent insert as suggested by Fig. 2C. As shown in Fig. 2A for example, a fastening system may comprise a fastening component 110, having fastening elements 114 which may be arranged in a micropattern 120. At least a portion of fastening elements may comprise integrally formed hooks. The fastening system may further comprise a receiving component 112 (see, e.g. Figs. 2A-2B), operatively engageable with the fastening elements. These and other features will be further described below.

Outer Cover

Fig. 2A depicts an exemplary outer cover 20 as it may appear opened and laid flat. In Fig. 2A, the wear-facing surface 22 of the outer cover 20 face the viewer. The outer cover comprises a front lateral edge 13, a rear lateral edge 19, and two longitudinal edges 17. The outer cover comprises a length, Lc, from the outboard most portion of the front lateral edge to the outboard-most portion of the rear lateral edge. The outer cover may comprise a lateral axis 23, dividing evenly its length, and a longitudinal axis 21. The longitudinal edges 17 may be parallel to the longitudinal axis 21. However, for better fit, longitudinal edges 17 may be curved or angled to produce, for example, an "hourglass" shape article when viewed in a plan view as shown in Fig. 2A, for example. In nonlimiting examples, the outer cover may be asymmetric, for instance having a width its rear region that is greater than the width in its front region. Thus, the width of the outer cover, Wcv, as measured between the longitudinal edges along a line parallel to the lateral axis, may vary along the longitudinal length of the outer cover. Nonlimiting examples of outer covers are disclosed in U.S. Pat. Nos. 9,387,138 and 8,435,223.

If it is desired that outer cover 20 be reusable, for outer cover 20 to remain substantially sanitary and useful (without requiring laundering or disposal) after removal and replacement of an insert, it may be desired that all parts of outer cover 20 remain substantially unsoiled after an exudation of waste (especially fecal matter) by the wearer. Thus, it may be desired that when insert 30 is installed within an outer cover 20, there is no non-removable portion or component of outer cover 20 that lies over or covers a substantial portion of wearer-facing surfaces of insert 30. Expressed differently, no non-removable portion or component of outer cover 20 is situated between a substantial portion of insert 30 and the wearer when the wearable absorbent article is worn, at least in the areas proximate to wearer body features that discharge exudates. Thus, it may be desired that outer cover 20 include no non-removable cover sheet or the like that covers or contains substantial portions of wearer-facing surfaces of insert 30 within outer cover 20, nor any overlying structures such as pockets, straps or flaps that substantially wrap or cover the insert proximate to exudate discharge points, or lie substantially between insert 30 and the wearer's anus and/or genitals, when the wearable absorbent article is worn. If outer cover 20 lacks such overlying structures, this may increase the likelihood that the wearer's exudates will contact only insert 30, and not portions of outer cover 20.

Outer cover 20 and/or layers or portions thereof may be made of any durable or semi-durable knitted, woven or nonwoven textile or textile-like material that is appropriately compatible with skin of the intended wearer(s). Suitable examples are described in U.S. Applications Ser. Nos. 12/687,493; 12/687,412; 12/687,528; and 12/687,425 (all by Roe et al.).

Non-limiting examples of fibers, nonwovens and laminates of nonwovens and films that might be considered for use as semi-durable outer cover materials may be found in U.S. Patents Nos. 7,223,818; 7,211,531; 7,060,149; 6,964,720; 6,905,987; 6,890,872; 6,884,494; 6,878,647; and 5,518,801; and U.S. Published Applications Nos. 2008/0319407; 2008/0045917; 2007/0293111; 2007/0287983; 2007/0287348; 2007/0249254; 2007/0203301; and 2005/0164587.

Fig. 2C depicts an exemplary outer cover, with its garment-facing surface 24 facing the viewer. In nonlimiting examples, the garment-facing surface 24 of the outer cover may be formed by a first layer of a durable or semi-durable material. The material selected may include fibers having hydrophobic properties, providing enhanced liquid containment attributes to such first layer. In another example, however, it may be desirable in some circumstances for the selected material to include hydrophilic fibers, or fibers treated to be hydrophilic, so as will cause the material to more readily absorb and/or transmit liquid therethrough. This may serve to provide supplemental absorbency within the outer cover for the event in which liquid exudates escape the insert, or to provide one way of communicating to the user that liquid exudates have escaped the insert. Additionally, in some circumstances, it may be desirable that the material selected have soft tactile properties so as to have a pleasant feel that the user and/or wearer find attractive. The material also may be selected so as to have a desired appearance, including but not limited to coloration, sheen, and/or texture.

Outer cover 20 may be formed of a single layer of a durable or semi-durable material, or may have two or more layers in the front region 14 and/or rear region 18. Accordingly, referring to Fig. 2A, in nonlimiting examples, a wearer-facing surface 22 of the outer cover may be formed by a second layer of a durable or semi-durable material. The material selected may include fibers having hydrophobic properties, providing enhanced liquid containment attributes to the second layer. In another example, however, it may be desirable in some circumstances for the selected material to include hydrophilic fibers, or fibers treated to be hydrophilic. This may be desired in some circumstances to cause the material forming inner surfaces 22 to more readily absorb liquid, or transmit liquid therethrough. This may serve to provide supplemental absorbency within the outer cover for an event in which liquid exudates escape the insert, reducing the likelihood that the outer cover will leak. Alternatively, it may provide one way of communicating to the user that liquid exudates have escaped the insert, by causing wetness to be transmitted through to the outer cover outer layer such that wetness is visible on outer surfaces. Alternatively, it may serve to provide a layer that tends to draw moisture away from the skin, for a drier, more comfortable feel.

Additionally, in some circumstances, it may be desirable that the material(s) selected for inner surfaces 22 have soft tactile properties so as to have a pleasant feel against the skin, particularly in areas where no portion of an insert is expected to be present between the outer cover and the wearer's skin. Further, it may be desirable that at least a portion of the inner surface comprise a material (e.g., comprising loops) that is engageable by hook fastening components. Additionally, or alternatively, a second layer of material may be formed of a textile material having enhanced elasticity, such as by inclusion of fibers of an elastomeric material (such as spandex). In another example, an intermediate film layer may be included, laminated or not laminated with another layer.

In addition to forming differing layers of differing materials, it may be desirable to form a single layer of differing materials, for example, differing materials in the respective front, crotch and/or rear regions of the outer cover. Such differing materials may be joined at a seam such as inner seam 25 and/or outer seam 26. For example, the material predominately forming the inner surface of rear region 18 may be selected primarily for its elasticity features, which may better serve to provide snug fit about wearer body contours and accommodate wearer movement (*i.e.,* about the buttocks and hips). By comparison, the material predominately forming the inner surface of front region 14 and/or crotch region 16 might be selected primarily for its hydrophobicity or hydrophilicity, which may better serve to contain liquid exudates.

Layers or other elements of the outer cover may be joined to each other via any suitable mechanism, including, for example, adhesives, mechanical bonding, ultrasonic bonding, sewing, stitching, serging, edging, and the like.

Waist Features, Leg Gasketing Systems, Ears

Referring again to Figs. 1-2C, the article and outer cover may comprise one or more waist features 27. The waist feature may be elastic and thereby provide better fit about the waist of the wearer. Elasticized waist features include waistbands, waist cuffs having pockets formed from a portion of the waist feature that is unattached from waist panels and/or belts, and/or other article components designed to fit securely about the abdomen of the wearer. Nonlimiting examples of elasticized waist features are disclosed in U.S. Patent App. Nos. 14/533,472; 15/074,675 and 62/855,001. Elasticized waist features may comprise one or more nonwoven or textile layers, which may be layers of the outer cover or discrete portions, and one or more elastic elements 28. In nonlimiting examples, the elasticized waist feature comprises elastic strands joined to the nonwoven and/or textile layer(s). In further nonlimiting examples, the elasticized waist feature comprises a laminate of one or more textile and/or nonwoven layers and one or more films. As shown in Fig. 2C, the elasticized waist element may comprise one or more rugosities 29 if the elastic material is strained prior to lamination. In other nonlimiting examples, the layers of the elastic laminate may be joined at zero applied strain and subsequently activated.

In alternative embodiments, the waist feature may be inelastic. In such configurations, the waist feature may provide additional anchoring about the waist of the wearer.

Additionally, or alternatively, the article may comprise a leg gasketing system 50, portions of which may be formed by the outer cover and/or the absorbent insert. The outer cover may include one or more elastic elements 28, such as films or elastic strands, extending through all or a portion of the leg opening 51 to form a leg band portion 52. The elastic elements may be laminated with one or more nonwoven layers and/or one or more textile layers. As described with respect to the waist feature, the leg band portion may include rugosities 29 as result of the lamination process. In other nonlimiting examples, the leg band portion may be a zero strain, activated laminate.

The waist features and/or leg band portions may be disposed along the edge of the outer cover, and in some circumstances, it may be desired to have elasticized waist and leg band portions situated along substantially the entire length of the leg and/or waist openings so as substantially or completely encircle the wearer's legs and/or waist while outer cover 20 is worn. The gathered material within rugosities 29 can serve to accommodate stretching of waist feature and leg band portions. This arrangement not only may provide for better fit about the wearer's legs, but also may enable the outer cover 20, when formed of appropriately sized and shaped material, to form a pouch-like structure 53 in the crotch region (see Fig. 1) when worn, which may serve to provide space within the outer cover to accommodate the insert 30 and help hold it in place within outer cover 20, in a substantially laterally centered position within the crotch region. This may be deemed advantageous in examples in which an insert is attached within outer cover 20 by fastener components only located proximate to the respective ends of insert, and not at any longitudinally intermediate locations, as described further below. Alternatively, or additionally, the elastic strands or strips in waist features 27 and leg band portions 52 may be affixed within the outer cover only at or near their respective ends, *e.g.*, within a pouch, tube or envelope structure formed of outer cover material -referred to herein as a "drawstring elastic". This will allow the elastic material and associated outer cover material to stretch and move freely and independently of each other, which may promote fit and comfort. A snug fit about the wearer's legs provided by such elasticized leg band portions 52 may serve to enhance containment capability of the wearable absorbent article. The outer cover 20 may also include anchoring supplements, bands or systems thereof as described in more detail in U.S. Patent No. 8,932,273.

The outer cover may include ears 70 in one or both of the front and region regions. As shown in Figs. 1 and 2A, the ears may include fastening components 110 such that the ears can be secured to the opposing region (e.g., rear ears may include fastening components that may engage with the front region). The outer cover may include receiving components 112 to operatively engage with fastening components 110. In nonlimiting examples, a fastening component 110 is disposed on a wearer-facing surface 22 of the outer cover and a receiving component 112 is disposed on a garment-facing surface 24. Additionally, or alternatively, the outer cover 20 may have disposed thereon one or more insert fastening components 110I, which may be operatively engageable with one or more insert receiving components 112I located on the absorbent insert as illustrated in Figs. 2A-2B. It may, however, be desirable to locate fastening components on the absorbent insert (also illustrated in Figs. 2A-2B), particularly when the insert is disposable, so that the fastening elements do not warp or weaken due to continued reuse and/or laundering.

Absorbent Insert

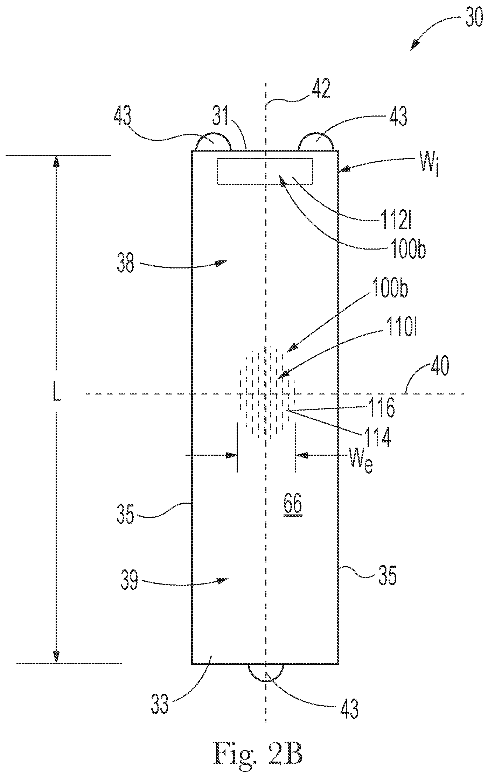

Returning to Fig. 2B, the absorbent insert 30 may be designed to contain and/or absorb body exudates, and may be made of pliable materials as will be described further below. The insert 30 includes a forward region 38 and a rearward region 39, a front edge 31 and a rear edge 33, two longitudinal sides 35, a lateral axis 40 and a longitudinal axis 42. The insert 30 comprises a length L from the outboard most portion of front edge 31 to the outboard most portion of the rear edge 33. The lateral axis equally divides the length L. In nonlimiting examples, the insert length L is less than the length of the outer cover Lc.

The longitudinal sides 35 may be generally parallel to the longitudinal axis. Alternatively, the longitudinal sides 35 may be curved, such as in an hour-glass configuration. Thus, the width of the insert, Wi, may vary. In nonlimiting examples, the width of the insert, Wi, is less than the width of the outer cover, Wcv, at one or more longitudinal positions. The width of the insert, Wi, may be less than the width of the outer cover, Wcv, throughout the length of the insert.

The insert also may include one or more grasp structures 43, such as forward and rearward grasp structures. The grasp structures may be provided to enable the user to quickly and easily grasp the insert, handle the insert during application and/or properly place the insert. In certain embodiments, the grasp structure may have a different tactile feel than surrounding or adjacent areas to distinguish the area and ease the user's identification of the grasp structures. The tactile feel of the grasp structure may be provided via fastening elements 114, such as integral fastening elements, and/or protrusions extending above or below the x-y plane of the substrate forming the grasp structure.

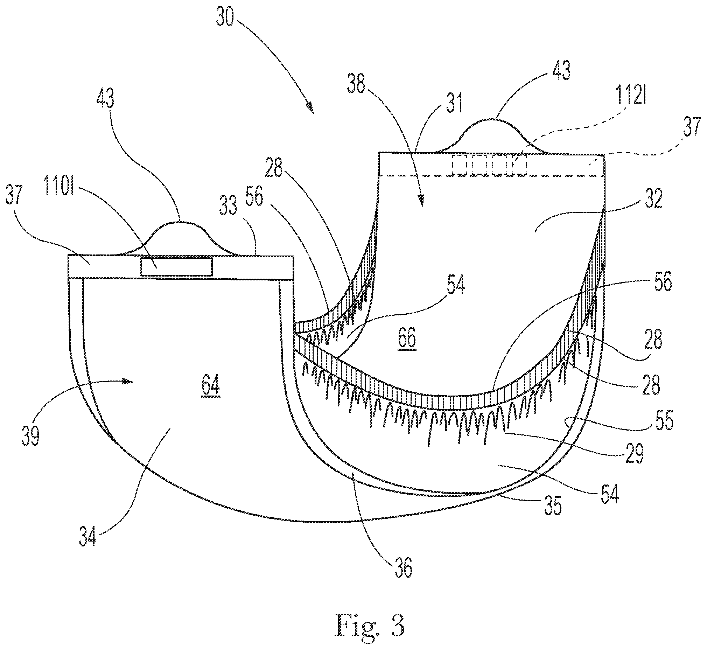

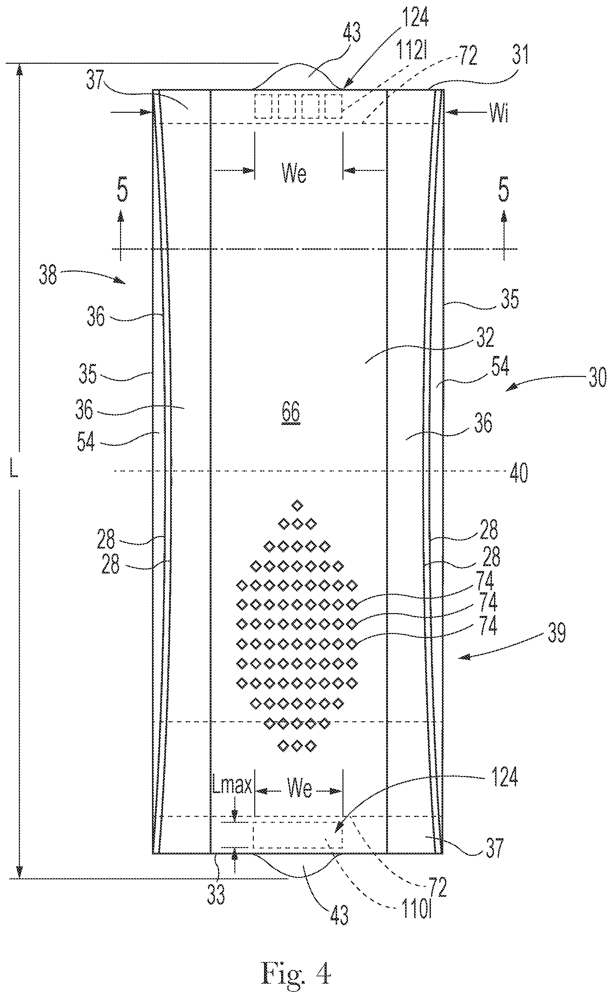

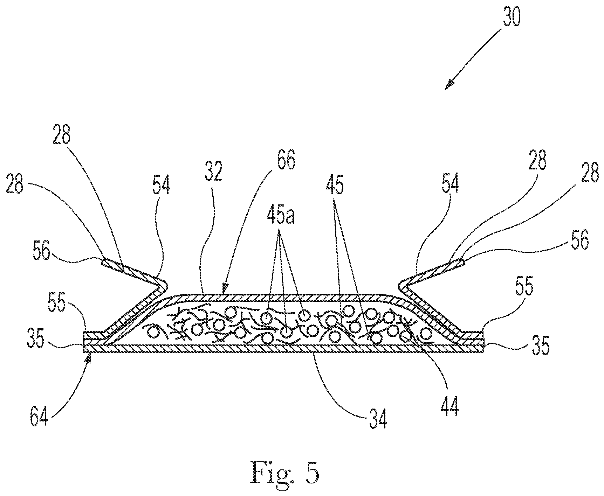

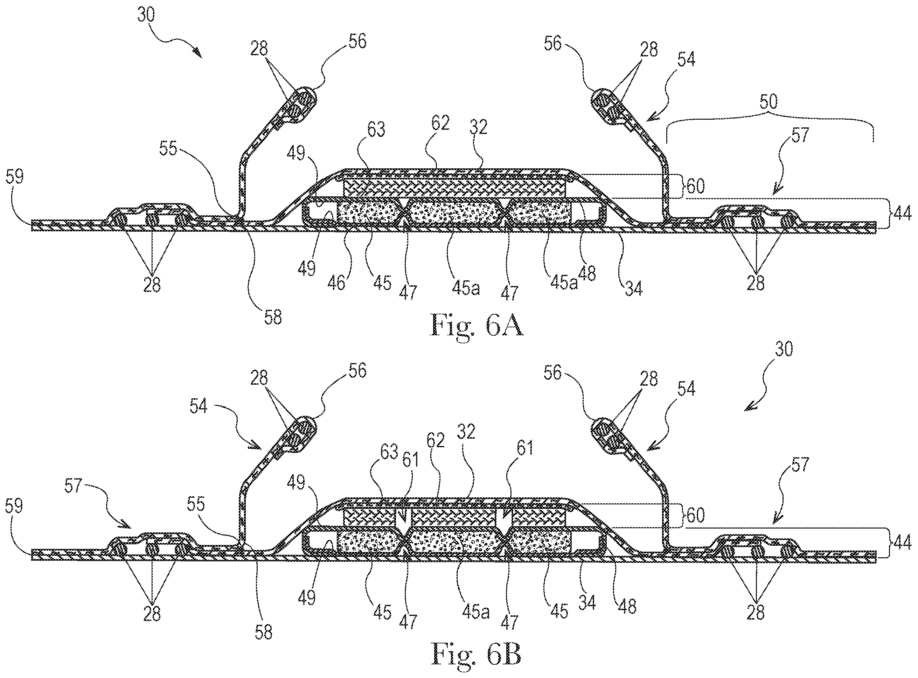

Fig. 3 depicts a disposable absorbent insert 30 shown in perspective view as it might appear in a free-standing, relaxed state. Fig. 4 depicts an example of an insert 30 shown stretched out and laid flat (against elastic-induced contraction to a position similar to that shown in Fig. 3), body-facing surfaces 66 facing the viewer. Fig. 5 depicts an exemplary cross-section of Fig. 4 according to certain nonlimiting examples. Figs. 6A-6B depict lateral cross sectional views of examples of inserts having a dual leg gasketing system, taken through a lateral axis of the insert example.

As shown in Figs. 3-4, the insert 30 may have a topsheet 32 and a backsheet 34. The topsheet and backsheet may be joined together along longitudinal seams 36 and along lateral seams 37. An absorbent core 44 may be disposed between the topsheet and the backsheet as shown for example in Fig. 5.

Returning to Figs. 3-4, the insert 30 may further include longitudinal standing cuffs 54 affixed along the longitudinal sides 35. The insert may also include insert fastening components 110I and/or insert receiving components 112I in the forward and/or rearward regions and/or at the lateral axis 42. Nonlimiting examples of absorbent inserts and details of their features are disclosed in U.S. Pat. Nos. 8,546,641 and 9,011,402.

It may be desirable to include a stiffening component 72 proximate to one or both ends of the insert 30, but especially an end adapted with a singularized and relatively localized fastening location providing a force-decoupled arrangement, as is discussed below. Referring to Fig. 4, end support stiffeners 72 may be included. Such end support stiffeners may serve to aid the user in engaging the insert with the outer cover and to help insert 30 maintain its intended shape and configuration while being worn beneath an outer cover (*i.e.,* help maintain the intended position and gasketing function of the standing cuffs 54 which are discussed below). End support stiffeners 72 also may help control the corners of the insert regardless of the size, type or location of fastener components included on the insert. End support stiffeners 72 may provide resistance to bending in any direction or plane. Further, because the ends of insert 30 may otherwise be folded over or bunched while being stored and/or carried by the user before installation, end support stiffeners may enhance user convenience, by causing the ends of insert 30 to maintain or seek a shape/configuration that requires less manipulation by the user to install it in an outer cover.

Topsheet

The topsheet 32 is generally a portion of the absorbent article 10 that may be positioned at least in partial contact or close proximity to a wearer. Suitable topsheets 32 are generally supple, soft feeling, and non-irritating to a wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured, may have any suitable three-dimensional features, and/or may have a plurality of embossments (e.g., a bond pattern). The topsheet 32 may comprise one or more apertures 74. The topsheet may be apertured by overbonding a material and then rupturing the overbonds through ring rolling, such as disclosed in U.S. Patent No. 5,628,097, to Benson et al., issued on May 13, 1997 and disclosed in U.S. Pat. Appl. Publication No. US 2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the top sheet.
Topsheet 32, backsheet 34 or any portion of the topsheet or backsheet may be embossed and/or matte finished to provide a more cloth-like appearance.

Backsheet

Backsheet 34 is generally the outer liner portion of insert 30 forming the garment-facing surface 64 thereof, and prevents the exudates absorbed and contained within insert 30 from wicking through and soiling the outer cover.
The backsheet 34 may comprise one or more nonwovens, elastomeric films, foams, strands, or combinations of these or other suitable materials with nonwovens or synthetic films. In nonlimiting examples, the backsheet is a laminate of an elastomeric material, such as a film, and a nonwoven.
In certain embodiments, the backsheet 34 is substantially water-impermeable. The backsheet may, for example, be or comprise a thin plastic film, such as a thermoplastic film having a thickness of about 0.012 mm to about 0.051 mm. Other suitable backsheet 34 materials may include breathable materials that permit vapors to escape from the absorbent article 10 while still preventing exudates from passing through the backsheet 34.
In certain embodiments, the backsheet 34 may have a water vapor transmission rate (WVTR) of greater than about 2000 $g/24h/m^2$, greater than about 3000 $g/24h/m^2$, greater than about 5000 $g/24h/m^2$, greater than about 6000 $g/24h/m^2$, greater than about 7000 $g/24h/m^2$, greater than about 8000 $g/24h/m^2$, greater than about 9000 $g/24h/m^2$, greater than about 10000 $g/24h/m^2$, greater than about 11000 $g/24h/m^2$, greater than about 12000 $g/24h/m^2$, greater than about 15000 $g/24h/m^2$, measured according to WSP 70.5 (08) at 37.8 °C and 60% Relative Humidity. A higher WVTR may be desired in this particular application, since the insert backsheet 34 will not form the outer surface of the wearable article, as a conventional disposable diaper backsheet would, but rather, will be covered by the one or more layers of the outer cover material(s) - which themselves may act in some circumstances to reduce WVTR of the composite structure.
Other suitable materials and/or manufacturing techniques may be used to provide a suitable backsheet 34 including, but not limited to, surface treatments, particular film selections and processing, particular filament selections and processing.
Backsheet 34 may be joined to topsheet 32, absorbent core 44 or any other element of insert 30 by any suitable attachment mechanism known in the art. For example, the attachment mechanism may include a continuous line or layer of adhesive, a patterned layer of adhesive, or an array of separate lines, spirals, or spots of adhesive. One example of an attachment mechanism comprises an open pattern network of filaments of adhesive as disclosed in U.S. Patent No. 4,573,986. Other suitable attachment mechanisms include several lines of adhesive filaments which are swirled into a spiral pattern, as is illustrated by the apparatus and methods shown in U.S. Patents Nos. 3,911,173; 4,785,996; and 4,842,666. Adhesives which have been found to be satisfactory are manufactured by H. B. Fuller Company of St. Paul, Minnesota and marketed as HL-1620 and HL 1358-XZP. Alternatively, the attachment mechanism may comprise heat bonds, pressure bonds, ultrasonic bonds, dynamic mechanical bonds, or any other suitable attachment mechanisms or combinations of these attachment mechanisms known in the art.

It will be appreciated that the outer cover described above can be constructed of materials and construction so as to bear and sustain a majority of the structural loading generally imposed upon a disposable diaper, by stretching and accommodation of the wearer's anatomical features and body movements, and by absorption, swelling and added weight resulting from the wearer's exudations of waste. Thus, lesser requirements for structural strength of an insert might be present with use of such an outer cover, as compared with strength required of inside components of a disposable diaper. Therefore, an article such as described herein may include a disposable absorbent insert manufactured from materials that are different from those ordinarily used in the manufacture of disposable diapers, such as petroleum-derived materials, e.g., polyethylene and polypropylene. For example, a disposable absorbent insert having one or more of a topsheet, backsheet, standing cuffs and/or other components formed of products of wood, cotton, flax (linen), hemp, bamboo, or other cellulose fibers (e.g., paper), in addition to the materials identified above, is contemplated. If resistance to aqueous liquid penetration or substantial liquid impermeability is desired, e.g., for a backsheet and/or standing cuffs, a material formed of ordinarily hydrophilic fibers such as paper may be coated or impregnated with a hydrophobic material, such as a skin-compatible oil or wax, to impart the desired resistance to aqueous liquid penetration. Each of the materials forming the insert may be selected so as to be dispersible in water or an aqueous solution, flushable, biodegradable and/or compostable (preferably to an agriculturally usable humus or soil amendment).

Absorbent Core

Turning to Fig. 5, the insert 30 may have an absorbent core 44 disposed within the envelope-like structure formed by the topsheet 32 and backsheet 34. The absorbent core 44 may comprise a wide variety of liquid-absorbent materials 45 commonly used in disposable diapers and other absorbent articles. Examples of suitable absorbent materials include comminuted wood pulp, which is generally referred to as air felt creped cellulose wadding; melt blown polymers, including co-form; chemically stiffened, modified or cross-linked cellulosic fibers; tissue, including tissue wraps and tissue laminates; absorbent foams; absorbent sponges; superabsorbent polymers; absorbent gelling materials (AGM); or any other known absorbent material or combinations of materials. The absorbent core may have an absorbent capacity of at least about 120 g, or at least about 150 g, or at least about 200 g, or from about 120 to about 300 g, reciting for said range every 10 increment therein, as determined by the Centrifuge Retention Capacity Test Method herein. In certain embodiments, at least a portion of the absorbent core is substantially cellulose free and contains less than 10% by weight cellulosic fibers, less than 5% cellulosic fibers, less than 1% cellulosic fibers, no more than an immaterial amount of cellulosic fibers or no cellulosic fibers. It should be understood that an immaterial amount of cellulosic material does not materially affect at least one of the thinness, flexibility, and absorbency of the portion of the absorbent core that is substantially cellulose free. Among other benefits, it is believed that when at least a portion of the absorbent core is substantially cellulose free, this portion of the absorbent core is significantly thinner and more flexible than a similar absorbent core that includes more than 10% by weight of cellulosic fibers. The amount of absorbent material, such as absorbent particulate polymer material 45a present in the absorbent core may vary, but in certain embodiments, is present in the absorbent core in an amount greater than about 80%, or greater than about 85%, or greater than about 90%, or greater than about 95% by weight of the core. In nonlimiting examples, a thermoplastic material, such as a thermoplastic adhesive composition 46, may be used to immobilize superabsorbent particles on a substrate (e.g., the topsheet, backsheet or core wrap).

In some embodiments, as shown in Fig. 6A, the absorbent core may comprise one or more channels 47, wherein said channels are substantially free of absorbent particulate polymer material. The channels 47 may extend longitudinally or laterally. The absorbent core may further comprise two or more channels. The channels may be straight, curvilinear, angled or any workable combination thereof. In nonlimiting examples, two channels are symmetrically disposed about the longitudinal axis.

The absorbent core 44 may include a core wrap 48, comprising one or more substrates 49 to encloses the absorbent material 45. Where channels are present, the core wrap may be bonded within one or more channels, thereby providing permanent channels which maintain their channel structure in the wet state.

The absorbent core 44 may be manufactured in a wide variety of sizes and shapes (*e.g.*, rectangular, hourglass, "T"-shaped, etc.). The configuration and construction of absorbent core 44 may also be varied (*e.g.,* the absorbent core(s) or other absorbent structure(s) may have varying caliper zones, hydrophilic gradient(s), a superabsorbent gradient(s), or lower average density and lower average basis weight acquisition zones; or may comprise one or more layers or structures). Exemplary absorbent structures for use as the absorbent core 44 are described in U.S. Patent No. 4,610,678; 4,673,402; 4,834,735; 4,888,231; 5,137,537; 5,147,345; 5,342,338; 5,260,345; 5,387,207; 5,397,316, and U.S. Patent App. Nos. 13/491,642 and 15/232,901.

Acquisition-Distribution System

Referring to Figs. 6A-6B, in some embodiments, an acquisition-distribution system (ADS) 60 is disposed between the topsheet 32 and the absorbent core 44. One function of the ADS is to quickly acquire one or more of the fluids and distribute them to the absorbent core in an efficient manner. The ADS may comprise one, two or more layers, which may form a unitary layer or may remain as discrete layers which may be attached to each other. The ADS 60 may include hydrophilic materials that provide significant wicking of bodily exudates. These materials may dewater the topsheet 32 and quickly move bodily exudates into the absorbent core 44. The ADS 60 may comprise one or more nonwoven materials, foams, cellulosic materials, cross-linked cellulosic materials, air laid cellulosic nonwoven materials, spunlace materials, or combinations thereof, for example. In some instances, portions of the ADS 60 may extend through portions of the topsheet 32, portions of the topsheet 32 may extend through portions of the ADS 60, and/or the topsheet 32 may be nested with the ADS 60. Typically, an ADS 60 may have a width and length that are smaller than the width and length of the topsheet 32. The ADS may have one or more channels 61 as described above with reference to the absorbent core 44. The channels 61 in the ADS may align or not align with channels 47 in the absorbent core 44. In an example, a first acquisition material 62 may comprise a nonwoven material and as second acquisition material 63 may comprise a cross-linked cellulosic material. Suitable ADS are described in WO 2000/59430, WO 95/10996, U.S. Pat. No. 5,700,254, and WO 02/067809, for example.

Cuff Structures

As noted above, the article 10 may comprise a leg gasketing system 50, portions of which may be formed by the insert 30. Returning to Figs. 3-4, the insert may comprise a pair of longitudinal standing cuffs 54, also referred to as barrier leg cuffs. Each standing leg cuff may be formed by a piece of material which is bonded to the absorbent insert so it may extend upwards from a wearer-facing surface and provide improved containment of fluids and other body exudates approximately at the junction of the torso and legs of the wearer. The standing leg cuffs are delimited by a proximal edge 55 joined directly or indirectly to the topsheet 32 and/or the backsheet 34 and a free terminal edge 56, which is intended to contact and form a seal with the wearer's skin. In some embodiments, the free terminal edge 56 comprises a folded edge. The standing leg cuffs 54 extend at least partially between the front edge 31 and the rear edge 33 on opposite sides of the longitudinal centerline 40 and are at least present in the crotch region.

The standing leg cuffs may be integral with the topsheet 32 or the backsheet 34 or may be a separate material joined to the topsheet and/or backsheet. Each standing leg cuff 54 may comprise one, two or more elastic elements 28 close to the free terminal edge 56 to provide a better seal. The standing cuff may be formed of any of a variety of substrates such as plastic films and woven or nonwoven webs of natural fibers (*e.g.,* wood or cotton fibers), synthetic fibers (*e.g.,* polyester or polypropylene fibers), or a combination of natural and synthetic fibers. In certain embodiments, the standing cuffs may be formed of a substantially liquid impermeable web to contain and isolate liquid exudates from the outer cover, outer clothing and environment of the wearer and/or may be formed of a vapor permeable web for breathability of the insert and article.

Referring to Figs. 6A-6B, the article 10 may comprise a dual gasketing system, which includes the standing cuffs and gasketing cuffs 57. The gasketing cuffs 57 may be joined to the insert 30, more particularly to the topsheet and/or backsheet. The gasketing cuffs are disposed outboard of the standing cuffs and may provide a better seal around the thighs of the wearer. A gasketing cuff 57 may comprises a proximal edge 58 and a free terminal edge 59. The free terminal edge 59 may comprise a folded edge. Each gasketing cuff may comprise one or more elastic elements 28, which may be sandwiched between other layers of material, such as the portions of material forming the attached proximal portions of the standing cuffs, topsheet, backsheet, separate gasketing cuff material, or combinations thereof. The gasketing cuff may be formed of any of a variety of substrates such as plastic films and woven or nonwoven webs of natural fibers (*e.g.,* wood or cotton fibers), synthetic fibers (*e.g.*, polyester or polypropylene fibers), or a combination of natural and synthetic fibers. As noted with respect to standing cuffs 54, gasketing cuffs may likewise be formed of any suitable web materials but preferably are formed of web materials that are effectively liquid impermeable while being vapor permeable, so as to contain the wearer's liquid exudates within the insert while permitting the insert to "breathe" to avoid excess humidity within the insert (which may overhydrate the wearer's skin and promote conditions such as diaper rash). Suitable materials include nonwoven, films, elastic strands and combinations thereof.

In further embodiments, the leg gasketing system comprises standing leg cuffs that are integral with gasketing cuffs. Suitable leg gasketing systems which may be part of the absorbent article are disclosed in U.S. Pat. App. No. 62/134,622, 14/077,708; U.S. Pat. Nos. 8,939,957; 3,860,003; 7,435,243; 8,062,279.

Fastening Systems

As noted above, the article 10 may comprise one or more fastening systems 100 as shown for example in Figs.

1-2C. A fastening system may be used to secure the outer cover to itself, for example, to close the article about the wear, to close the outer cover about the insert and/or to close the article for disposal. Additionally, or alternatively, a fastening system may be used secure the absorbent insert to the outer cover. In nonlimiting examples, the article 10 comprises at least two fastening systems, a first fastening system 100a to secure the article about the wearer and a second fastening system 100b to secure the outer cover to the absorbent insert.

In further nonlimiting examples, the article 10 includes a third fastening system 100c to further secure the article about the waist of the wearer. In such nonlimiting examples, the outer cover may comprise primary and secondary fastening systems (100a, 100c) having two distinct fastening locations, which deconcentrates lateral tensile focuses and reduces the tendency of the front portion of the article to pivot around the single fastening location. Suitable dual fastening systems are disclosed for example in U.S. Pat. App. Nos. 16/684,230 and 16/545,425.

The article may include a fourth fastening system 100d to secure the outer cover flap 76 to the topsheet and/or backsheet as indicated in Fig. 2C. Although named first, second, third and fourth for purposes for ease of understanding, it is to be understood that any combination of fastening systems is contemplated. Further, any number of fastening systems may be included to the extent workable.

Each system may comprise one or more fastening components 110 and one or more receiving components 112. A receiving component is operatively engageable with a fastening component. Nonlimiting examples of engageable fastening and receiving components include tape tabs, hook and loop fastening components, interlocking fasteners such as tabs & slots, buckles, buttons, snaps, and/or hermaphroditic fastening components. Some exemplary surface fastening systems are disclosed in U.S. Patent Nos. 3,848,594; 4,662,875; 4,846,815; 4,894,060; 4,946,527; 5,151,092; and 5,221,274. An exemplary interlocking fastening system is disclosed in U.S. Patent No. 6,432,098. The fastening component and/or the receiving component may further include a release tape or other material, including folded material, that protects the component from insult prior to use.

The fastening component 110 comprises one or more fastening elements 114 which cause the component to join with another surface, such as the receiving component. In various embodiments, fastening elements comprise hooks 116. Receiving component 112 comprises material adapted to fastenably cooperate with fastening elements, such as a section or patch adapted to serve as cooperative loops material, to provide a hook-and-loop fastening system combination. The fastening and/or receiving components may be discrete from and joined to the outer cover or absorbent insert or may be integral with one or both. In nonlimiting examples, nonwoven material (such as nonwoven material forming portions of the outer cover surfaces or portions of the insert backsheet or topsheet) may be comprise integral loops material 125 as illustrated in Fig. 2A. In further nonlimiting examples, fastening components and receiving components may be formed on the same patch of material. For example, a fastening component may be integrally formed from a patch of loops material joined to the absorbent insert or outer cover.

Fastening and receiving components may be disposed in various locations depending on the desired functionality of the fastening system. The outer cover 20 may comprise a fastening component on its wearer-facing surface 22, such as on the wearer-facing surface of its ear 70, and comprise a receiving component 112 on the garment-facing surface 24 of the opposing waist region as indicated in Figs. 1 and 2A. Additionally, or alternatively, the outer cover 20 may comprise a fastening component 110 on its garment-facing surface 24, such as on the garment-facing surface of the front waist region 14, and a receiving component on its inner surface 22, such as the inner surface of a rear ear 70.

The absorbent insert 10 may comprise one or more insert fastening components 110I on its garment-facing surface 66, and the outer cover 20 may comprise one or more insert receiving components 112I on its wearer-facing surface 22 as shown in Figs. 2A and 2B. Likewise, the outer cover may comprise a fastening component 110I for securing the absorbent insert 30 thereto. And, the absorbent insert may comprise a receiving component 112I. A fastening component and/or receiving component may be disposed outboard of the lateral and/or longitudinal axes of the outer cover and/or the absorbent insert. A fastening component and/or receiving component may at least partially overlap the lateral and/or longitudinal axis of the outer cover and/or the absorbent insert. This may be particularly helpful to assist the user in placing the absorbent insert centrally with respect to the outer cover.

Fastening and/or receiving components may be disposed along the longitudinal sides proximate the ends of the insert in the forward and/or rearward region of the insert. Fastening and/or receiving components may be disposed adjacent an edge of the insert, or may be disposed laterally and longitudinally inboard of the edge. For example, a fastening and/or receiving component may be disposed no more than about 50 mm from the front lateral edge 31, or no more than about 25 mm from the front lateral edge, or from about 5 mm to about 50 mm, or from about 10 mm to about 25 mm inboard of the front lateral edge, reciting for each range every 1 mm increment therein. A fastening and/or receiving component may be disposed no more than about 50 mm, or no more than about 25 mm, or from about 5 mm to about 50 mm, or from about 10 mm to about 25 mm inboard of the rear lateral edge 33, reciting for each range every 1 mm increment therein.

In various nonlimiting examples, at least about 50%, or at least about 75%, or at least about 90%, or substantially all of the wearer-facing surface of the outer cover is adapted to serve as an insert receiving component 112I (i.e., at least 50% etc. of the surface area of the wearer-facing surface is a receiving component). In this way, the placement of the absorbent insert is less limited by the receiving component position, or not at all limited in embodiments where substantially all of the outer cover surface comprises the receiving component.

It is also contemplated that the absorbent insert may comprise one or more insert receiving components 112I, and at least about at least about 50%, or at least about 75%, or at least about 90%, or substantially all of the garment-facing surface of the absorbent insert may be adapted to serve as an insert receiving component 112I.

In nonlimiting examples, the fastening component 110I may be smaller than the receiving component 112I. The fastening component 110I may comprise a length, Lmax, that about 50% or less, or about 40% or less, or about 30% or less, or from about 5% to about 50%, or from about 10% to about 30%, of the length of the insert receiving component to which it engages, reciting for each range every 5% increment therein.

The overall thickness 316 of the receiving component 112I can be from about 1 to about 1000 micrometers, or from about 350 micrometers to about 650 micrometers, reciting for each range every 20 micrometer increment therein, as measured by the Thickness Test Method herein. The insert receiving component may comprises a basis weight of about 10 gsm to about 100 gsm, reciting for said range every 2 gsm increment therein.

The insert receiving component may be formed from a fibrous material wherein the majority of fibers comprise a cross-sectional dimension of about 25 micrometers or less, or about 20 micrometers or less, or about 15 micrometers or less, or from about 5 micrometers to about 150 micrometers, reciting for said range every 5 micrometer increment therein. The fibers may comprise any suitable cross-sectional shape, including circular, oval, squarish, rectangular, triangular, star-shaped, multi-lobal and combinations thereof. In nonlimiting examples, a majority of the fibers comprise a cross-sectional shape that is round. For fibers with non-circular cross-sections, the overall fiber cross-sectional dimension is the largest distance measured linearly across the cross-section of the fiber. The overall fiber cross-sectional dimension of a fiber can be determined by making measurements on an enlarged image of the fiber, taken with equipment, at high magnification. For example, a Scanning Electron Microscope can be used.

The insert receiving component may be formed from any suitable material including a nonwoven, woven, polyester, nylon and combinations thereof.

The insert fastening and receiving component combination may comprise a CD Shear Strength of about 40 N or less, or about 20 N or less, or about 10 N or less, or about 5 N or less, or about 3 N or less, or from about 2 N to about 40 N, according to the Dynamic Shear Strength Test Method herein, reciting for said range every 1 N increment. Additionally, or alternatively, the insert fastening and receiving component combination may comprise a CD Peel Strength of about 3 N or less, or about 2 N or less, about 1.5 N, or about 1 or less, or about 0.5 or less, or from about 0.2 to about 3N, reciting for said range every 0.25 N increment therein, according to the Peel Strength Test Method herein. Without being bound by theory, it is believed the thickness, basis weight, fiber cross-sectional dimension, shear strength and/or peel strength cited herein facilitates the fastening system effectively retaining the insert to the outer cover in the proper position relative to the wearer's anatomy during use with being overly abrasive to the outer cover or causing tearing during removal.

Fastening and/or receiving component may be discrete from the outer cover or absorbent insert, or may be integral with the outer cover or absorbent insert. Any workable arrangement of the fastening component and receiving components is within scope of the present invention. Additional details of fastening and receiving components are discussed below

Additionally, or alternatively, a fastening system may form a singularized and localized fastening location proximate each of, or either, the forward/front and/or rearward/rear ends/regions. Having only two respectively singularized, localized fastening locations provides an advantage of simplicity and ease of installation for the user, by presenting only two locations at which the user must co-locate and fasten the absorbent insert 30 to the outer cover 20. Additionally, having a single, localized fastening location proximate only one or two ends of insert 30 provides a partially force-decoupled attachment of the insert 30 within the outer cover 20. This may be especially desired in the rear region 18 of outer cover 20, where extensibility and/or elasticity of outer cover 20 may be especially desired to maintain fit, comfort and appearance while accommodating body contours and movements of the wearer about the buttocks and hips. With this force-decoupled arrangement, the portions of outer cover 20 surrounding fastening element (s) 110, such as waistband portions 27, and front and rear regions 14, 18, may laterally stretch and contract substantially independently of and unimpeded by the structure of insert 30 and portions thereof.

Thus, in one example, referring to Fig. 4, a fastening system comprises a cover-insert engagement area 124 laterally centered on the insert as suggested in the figure, and may have an engagement width We (i.e., the maximum width of the fastening and receiving pair when joined) that extends no more than about 50% of the lateral width $W_i$ of the insert 30 at forward region 38 and/or rearward region 39. This provides that no more than about 50% of the width of the insert 50 at, e.g., forward region 38 and/or rearward region 39, will be force-coupled to the outer cover by operation of the fastening and receiving components. In some circumstances, it may be desired that the portion of the lateral width of forward region 38 and/or, especially, of rearward region 39 of insert 50 that is force-decoupled from the outer cover is about 50% or greater, or about 60% or greater, or about 70% or greater, or about 80% or greater, or from about 40% to about 95%, or from about 50% to about 90% of the lateral width of the insert at any longitudinal location when installed therein, reciting for each range every 5% increment therein.

It is also contemplated, however, that the engagement area 124 cover more than about 50%, or more than about 75%, or even about 100% , or from about 60% to about 100% of the lateral width of the insert at the longitudinal location where the fastening component is disposed or engaged. In this way, the absorbent insert would attach from edge to edge, ensuring a sustained attachment and/or aiding in the absorbent insert being smoother and wrinkle free during wear.

The fastening component may have a maximum longitudinal length, $L_{max}$, that is less than the longitudinal length of the component on which the fastening component is disposed. For example, if the fastening component is disposed on the absorbent insert, its length, $L_{max}$, may be less than the length, L of the insert. If the fastening component is disposed on the outer cover, its length, $L_{max}$, may be less than the length, Lc, of the outer cover. Likewise, the fastening component may have a maximum width that is less than the width of the absorbent insert, Wi, and/or less than the width of the outer cover, Wcv, in the area where the fastening element is present. In various nonlimiting examples, however, the fastening component may have a maximum width that is substantially equal to the width of the component to which the insert is attached.

In some embodiments, the fastening component 110 may be separately applied sections or patches of fastening elements that are bonded to absorbent insert or chassis by heat, compression, adhesive, ultrasonic bonding or any combination thereof.

In other examples, a fastening component may be a plurality of integrally formed fastening elements 114, such as fastening elements that are formed directly on a section of the outer cover or absorbent insert, or more particularly formed directly on a section of a polymeric layer of nonwoven. For example, fastening hooks 116 may be produced via application of molten polymer resin onto the layer, and subsequent formation of hooks in and from the melted, applied resin via known methods. The fastening components may be integrally formed from polymeric material by heating and softening a portion of the material and pressing it into hook-forming cavities, as is disclosed in U.S. Pat. No. 8,784,722. The fastening components may be integrally formed from the polymeric material through a single continuous process as is disclosed in commonly assigned U.S. Pat. App. No. 16/545,425, under attorney docket 15308M. Hooks-forming cavities may be formed and arranged on a hooks-forming roller in any desired configuration of hook size, shape, number, density, placement pattern, and arrangement of areas of hooks.

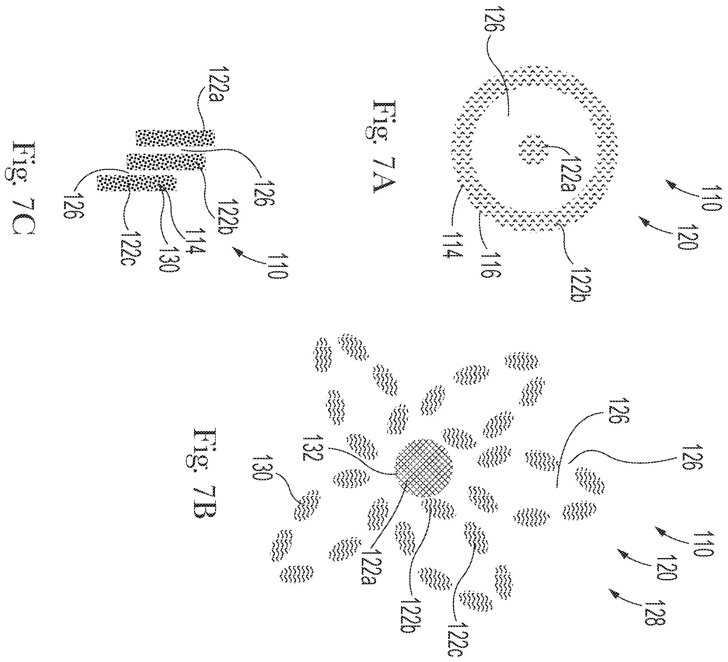

Using the molding process described above, the practical constraints and/or costs presented by supply and application of a continuous strip of pre-manufactured hooks material are eliminated, and the areas of fastening elements may be provided on the nonwoven material in any desired configuration, such as the configurations reflected in Figs. 7A-7C. It can be appreciated that areas of fastening elements may be configured in any desired size, shape, pattern, directionality of hooks, number of hooks or orientation. An orientation of an area of hooks is the angle of a line passing through the maximum dimension of the area with respect to the longitudinal axis of the article component.

By way of nonlimiting examples, areas of fastening elements may be disposed in lines, rectangular shapes and/or shapes formed from curvilinear sections. Areas of fastening elements may be configured as discrete, discontinuous shapes entirely surrounded by areas not occupied by fastening elements, as may be seen in Figs. 7B-7C (sometimes known as "islands-in-the-sea" configurations). Continuous areas of fastening elements may be configured to entirely surround discrete, discontinuous shapes of areas not occupied by fastening elements, as may be seen in Figs. 7A

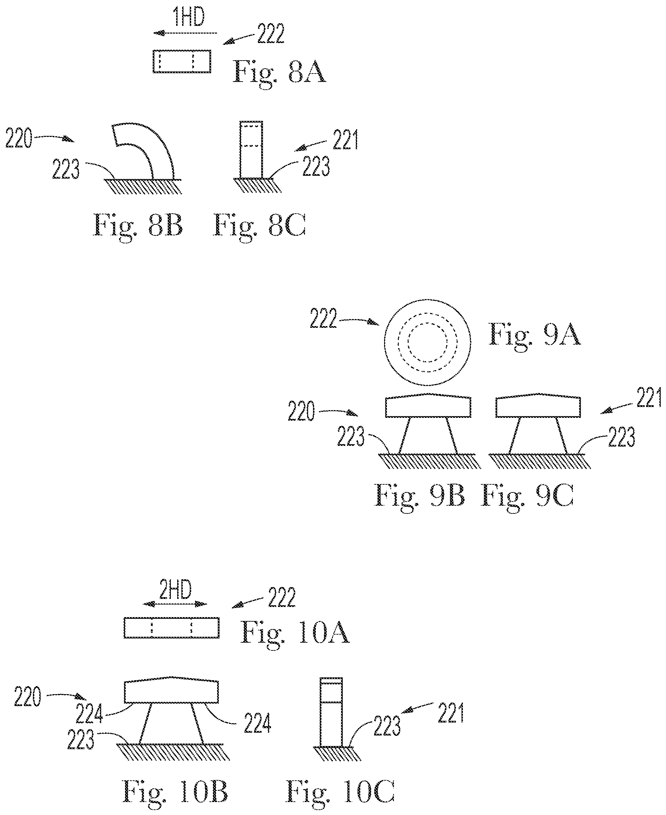

Further to the above, integral fastening elements may be formed with varying directionality to provide different benefits in different sections of the component. For instance, hooks which are asymmetric about their vertical centerline (e.g., the inverted J-shape shown in Fig. 8B or similar hook configuration) may be formed so that the open portion is pointed in the direction of expected engagement. In further nonlimiting examples, hooks in a front waist region 14 may be imparted with directionality approaching or along the lateral direction and extending toward the longitudinal axis of the diaper. Such directionality provides mechanical structure extending in a direction opposite the ordinary direction of shear forces (directed away from the longitudinal axis in the front region) that would be exerted on the hooks in that region while the hooks are engaged during wear, providing for added fastening strength and/or more secure attachment, as compared with non-directional hooks of similar size, material utilization (shape volume) and numerical density. Hooks in the rear waist region may be imparted with directionality toward the longitudinal axis of the diaper (when the fastening member is in the open position). Such directionality would oppose the ordinary direction of shear forces that would be exerted on the hooks in the front waist region when the hooks are engaged (i.e., fastened) during wear, providing for added fastening strength and/or more secure attachment, as compared with non-directional hooks of similar size, material utilization (shape volume) and numerical density.

Other hook shapes are shown in Figs. 9A-10C. Each of Figs. 8A-10C depicts a front view 220, side view 221 and top view 222 of one of three non-limiting examples of hook shapes, protruding or emerging from a substrate 223. (Substrate 223 may be the material 140 as described below, from which the hooks are integrally formed.) The hook shape example reflected in Figs. 8A-8C is substantially unidirectional in that it hooks over predominately in one direction 1HD. However, as described below, the direction hooks in one array may be different from the direction in another array or another area of the article. Referring to Figs. 9A-9C, this type of hook shape (sometimes described as a "mushroom" shape) lacks directionality because it is substantially symmetrical about all planes along its vertical (z-direction) axis and/or has substantially similar front and side view profiles. Other types of hook shapes may be formed to have directionality such that they lack such symmetry and/or similarity of front and side views. The hook shape reflected in Figs. 10A-10C (sometimes described as an "arrowhead" shape) is substantially bi-directional in that it has two opposing arms 224 that hook over in two opposite directions 2HD.

The flexibility in the above-mentioned process permits more precise placement of fastening elements to effectuate certain properties (e.g., greater bond strength) in certain areas. Returning to Figs. 7A-7C, in certain embodiments, the fastening elements may be disposed in a macro pattern 120 having a plurality of arrays 122, including a first array 122a and a second array 122b of fastening elements. The macro pattern may optionally include additional arrays, such as a third array 122c. An array of fastening elements is a plurality of fastening elements wherein each element is no more than about 2 mm, or from about 0.1 mm to about 2 mm, or from about 0.5 mm to about 1.5 mm from at least one of the other elements in the plurality, reciting for each range every 0.1 mm increment therein. A macro pattern comprises at least two arrays of fastening elements, wherein each array is spaced from at least one other array by 2 mm or more, or about 2.25 mm or more, or about 35 mm or less, or about 30 mm or less, or about 25 mm or less, or about 15 mm or less, from about 2.25 mm to about 35 mm, or from about 3 mm to about 30 mm, or from about 2.5 mm to about 25 mm, or from about 3 mm to about 20 mm, or from about 4 mm to about 15 mm, reciting for each range every 1 mm increment therein. A void area 126 (i.e., areas free of fastening elements) separates the arrays. Arrays of fastening elements, or a macro pattern, may form a line, a curve, a geometric shape, a non-geometric shape, design element 128 or may form a closed shape surrounding a region free of fastening elements 126. Spacing and shape of individual arrays within a pattern may be the same or different in different portions of the macro pattern. Arrays may differ from one another in any of the following characteristics: peel strength, the number of fastening elements, shapes of fastening elements, types of fastening elements (e.g., hooks, tabs), directionality of fastening elements, orientation of array, average spacing of fastening elements, whether the elements are discrete or integral or some combination, fastening element constituent materials (e.g., material from which the fastening elements are made (e.g., nonwovens, films and combinations thereof)), the number and/or types of layers from which integral fastening elements are formed, average size of the fastening elements, aggregate shape of the array, surface area, opacity, color and combinations thereof. A first array 122a may surround a second array 122b as indicated in Fig. 7A for example. The second array 122b may be disposed laterally inboard of the first array 122a, and/or longitudinally inboard of the first array or vice versa.

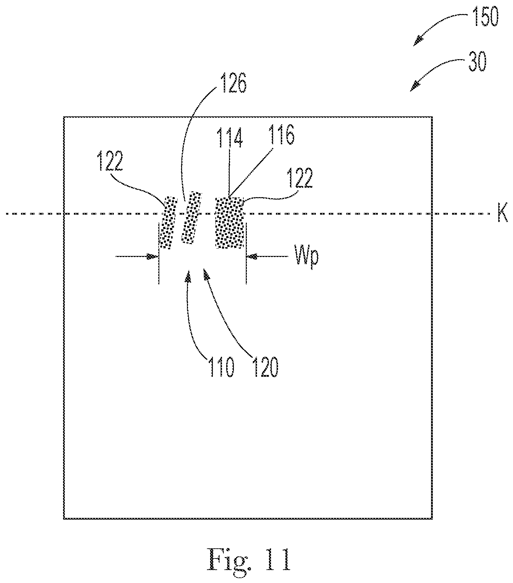

Turning to Fig. 11, a macro pattern may comprise a maximum width, Wp. The maximum width, Wp, is the distance between the laterally furthest apart fastening elements. The maximum width of the macro pattern, Wp, may be at least about 10% of the width of the article component 150 to which the fastening component is joined or integral with, along a line K extending through the longitudinal center of the macro pattern and being parallel to the lateral axis of the article. That is, if the fastening component 110 is disposed on with the absorbent insert, the macro pattern may extend at least about 10% of the width of the absorbent insert, Wi, at line K. If the fastening component 110 is disposed on the outer cover, the macro pattern may extend at least about 10% of the width of the outer cover, Wcv, at line K. It may be desirable that the macro pattern is at least about 20%, or at least about 40%, or at least about 50%, or at least about 60%, or from about 10% to about 100%, or from about 25% to about 75%, or from about 30% to about 60% of the width of the article component 150 at line K.

The article component 150 may comprise the outer cover 20 and/or the absorbent insert 30, or discrete materials joined to the outer cover and/or insert (e.g., discrete leg cuff materials, ears, waistbands, landing zone patches of material) and any combinations of the foregoing.

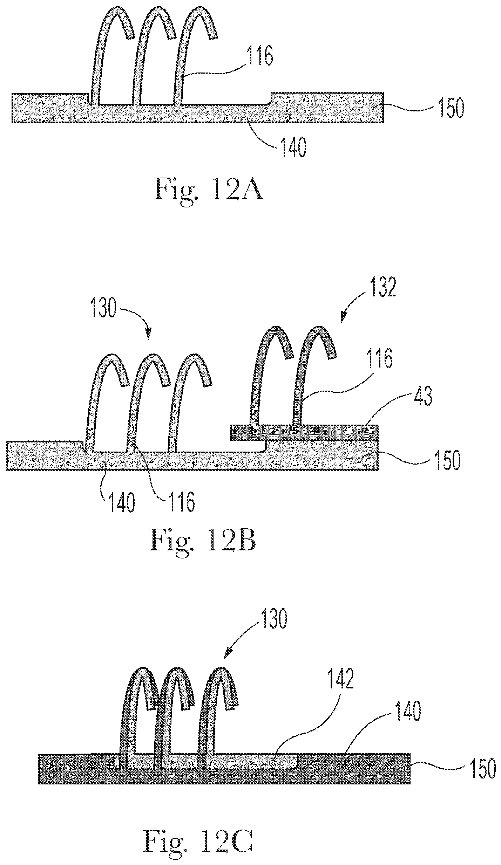

The macro pattern may comprise at least about 20%, or at least about 40%, or at least about 50%, or at least about 60%, or at least about 75%, or about 100% of integrally formed fastening elements 130 based on the number of fastening elements. Integral fastening elements 130 may be formed from one material layer 140 of the absorbent article component 150 as suggested in Fig. 12A, or from multiple material layers 140, 142 of the article component 150 as is suggested in Fig. 12C. In nonlimiting examples, the macro pattern comprises both integrally formed fastening elements 130 and discrete, non-integral fastening elements 132. For instance, the first array may comprise integrally formed hooks and the second array may comprise a discrete patch 143 of material having fastening elements joined to the article component 150, as suggested by Figs. 7B and 12B. Suitable material layers may include polymeric materials such as nonwovens, films and combinations thereof. Material layers may also be selected from elastomers, polyolefinic materials, adhesives, inks, dyes, tactile modifiers (e.g., silicone) and combinations thereof.

Differing arrays allows for differences in fastener properties at different locations. Two arrays may differ in peel strength, types of fastening elements, types of hook shapes (if applicable), directionality of fastening elements, aggregate shape, surface area, opacity, color, spacing of fastening elements, size of elements, number of fastening elements, constituent fastening element material, design element(s) and combinations thereof.

Where multiple fastening systems are provided on the article, they may differ in peel strength, macro patterns (including the presence or absence of a macro pattern), types of hook shapes (if applicable), directionality of fastening elements, size of fastening and/or receiving components, types of fastening elements (e.g., hooks, adhesive), types of receiving components, fastening component type (i.e., integral or discrete or combination), receiving component type (i.e., integral or discrete or combination), location of fastening and/or receiving components and combinations thereof. Where multiple fastening components each comprise macro patterns, the macro patterns may differ by peel strength, design element, surface area, opacity, color, array characteristics noted above, combination of arrays, spacing of arrays from one another, relative positioning of arrays, the number of arrays and combinations thereof.

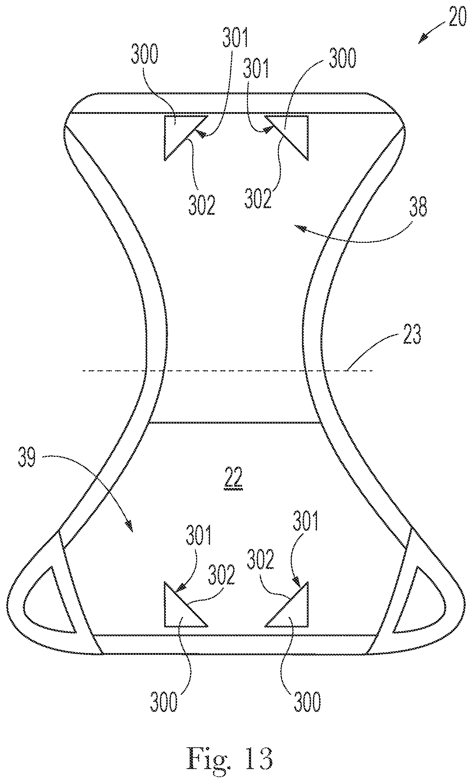

Further to the above, it is to be understood fastening systems need not necessarily include respective components of a two-component fastening system. Rather, a fastening system may require only one component. By way of nonlimiting example, a fastener component on outer cover 20 may include a patch of adhesive; a structure having a region of relatively high coefficient of friction; a pocket; flap; strap; or other capturing, holding and/or retaining surface, device or structure. Thus, a receiving component is unnecessary. Referring to Fig. 13, in some nonlimiting examples, the outer cover 20 may include one or more pocket structures 300 situated on or along the inner surface 22. A pocket structure may be adapted to receive, fit and capture, for example, the forward edge and a portion of forward region 38 of insert 30. A pocket structure 300 may have an opening 301 facing lateral axis 23, such that an end of insert 30 may be inserted therein and retained thereby. A pocket structure may alternatively have an opening 300 facing away from lateral axis 23, such that an end of insert 30 may be inserted therein and retained thereby, and then insert 30 may be folded back over such opening and toward lateral axis 23. One or more pockets 300 may be provided in the front and/or back region on the inner surface of the outer cover 20 positioned such that a corner of an insert 30 may be inserted into and retained by the pocket. Such pockets may have respective openings defined by edges of material 302 forming an angle with respect to both the lateral and longitudinal axes of between about 0 and 90 degrees, but more preferably, between about 30 degrees and 60 degrees.

Bio-Sourced Materials

Components of the disposable absorbent article can at least partially be comprised of bio-sourced content as described in U.S. Pat. Pub. Nos. 2007/0219521A1, 2011/0139658A1, 2011/0139657A1, 2011/0152812A1, and 2011/0139659A1. These components include, but are not limited to, topsheets, backsheet films, backsheet nonwovens, side panels, leg gasketing systems, superabsorbent, acquisition layers, core wrap materials, adhesives, outer covers, fastener systems, and landing zones. In at least one embodiment, a disposable absorbent article component comprises a bio-based content value from about 10% to about 100%, or from about 25% to about 75%, or from about 50% to about 60% using ASTM D6866-10, method B. In order to apply the methodology of ASTM D6866-10 to determine the bio-based content of any component, a representative sample of the component must be obtained for testing. In at least one embodiment, the disposable absorbent article component can be ground into particulates less than about 20 mesh using known grinding methods (e.g., WILEY® mill), and a representative sample of suitable mass taken from the randomly mixed particles.

Test Methods

Hysteresis Test

The following test methods utilize a commercial tensile tester (e.g., from Instron Engineering Corp. (Canton, Mass.), SINTECH-MTS Systems Corporation (Eden Prairie, Minn.) or equivalent) interfaced with a computer. The computer is used to control the test speed and other test parameters and for collecting, calculating, and reporting the data. The tests are performed under laboratory conditions of 23 deg. C.+-2 deg. C. and relative humidity of 50%+-2%. The samples are conditioned for 24 hours prior to testing.

1. Select a 2.54 cm (width), 7.62 cm (length) sample of the material for testing. In some cases, if it is not be possible to get a 2.54 cm x 7.62 cm sample, a smaller sample may be used, but a gage length of 25 mm must still be used. If the sample is activated or includes an activation portion, the length of the sample is taken in the direction of activation.

2. Select the appropriate jaws and load cell. The jaws must have flat surfaces and must be wide enough to fit the sample (e.g., at least 2.54 cm wide). Also, the jaws should provide adequate force to ensure that the sample does not slip during testing. The load cell is selected so that the tensile response from the sample tested is between 25% and 75% of the capacity of the load cell used.

3. Calibrate the tester according to the manufacturer's instructions.

4. Set the distance between the grips at 25 mm.

5. Place the sample in the flat surface of the jaws such that the longitudinal axis of the sample is substantially parallel to the gauge length direction. Mount the sample with minimal slack. Set the slack preload at 0.02 N/cm. This means that the data collection starts when the slack is removed with a force of 0.02 N/cm. Strain is calculated based on the adjusted gauge length (lini), which is the length of the sample in between the grips of the tensile tester at a force of 0.02 N/cm. This adjusted gauge length is taken as the initial sample length, and it corresponds to a strain of 0%. Percent strain at any point in the test is defined as the change in length divided by the adjusted gauge length times 100%.

6(a) First cycle loading: Pull the sample to a strain of 50% at a constant cross head speed of 254 mm/min.

6(b) First cycle unloading: Hold the sample at 50% strain for 30 seconds and then return the crosshead to its starting position (0% strain) at a constant cross head speed of 254 mm/min. Hold the sample in the unstrained state for 1 minute.

6(c) Set from second cycle loading: Pull the sample at a constant cross head speed of 254 mm/min, till it reaches a load of 0.05 N/25.4 mm (0.020 N/cm). Record the extended gauge length (lext). Next, return the crosshead to its starting position (zero strain) at a constant cross head speed of 254 mm/min. Set is defined as the strain at a second cycle load of 0.05 N/25.4 mm (0.020 N/cm). Calculate % set as indicated below.

6(d) Second cycle unload: Next, return the crosshead to its starting position (zero strain) at a constant cross head speed of 254 mm/min.

Percent Set is defined as the percent strain at a second cycle load of 0.05 N/25.4 mm (0.020 N/cm). Calculate % set as indicated below.

A computer data system records the force exerted on the sample during the test as a function of applied strain. From the resulting data generated, the following quantities are reported (note that loads are reported as force divided by the width of the sample and do not take into account the thickness of the sample):

1. Loads at 25% strain and 50% strain (N/cm)
2. % set (Percent Strain measured at a second cycle load of 0.02N/cm);
3. 

$$\% \text{ set} = (\text{lext-lini}) \,/\, \text{lini} * 100\%.$$

Five repetitions are done on each sample and the average and standard deviation reported.

The Hysteresis Test can be suitably modified depending on the expected attributes and/or properties of the particular material sample to be measured. For example, the Test can be suitably modified where a sample of the length and width specified above are not available from the subject article.

Centrifuge Retention Capacity (CRC) Test

The Centrifuge Retention Capacity (CRC) is a measure of the fluid retention capacity of a specimen submerged in 0.9% NaCl saline solution for 30 minutes and then subjected to centrifugation. The test is based on the Worldwide Strategic Partners Standard Test Method WSP 241.2 (09). Gravimetric Determination of Fluid Retention Capacity in Saline Solution after Centrifugation.

For analysis, specimens are placed into a bag constructed from heat-sealable. water-permeable, non-apertured nonwoven. Specifications for the bag material are given in WSP 241.2 (09). Measure the length (L) and width (W) of the specimen to be tested. Cut a piece of the bag material that is 2×L+50 mm by W+25 mm. Fold the bag material in half across its width and heat-seal two of the open edges approximately 3 to 5 mm from the edges. The finished bag is L+25 mm by W+25 mm. In addition to the bags for samples, prepare three bags of the same dimensions to be used as blanks.

Absorbent Insert Sample Preparation: Precondition samples at about 23° C.±2 C.° and about 50%±2% relative humidity for 2 hours prior to testing. The absorbent insert is unfolded and placed with the wearer-facing surface facing upward. Using scissors cut any elastic along the longitudinal edges of the article at an interval of approximately 2 cm, such that the article can be laid flat. Lay the absorbent insert on a piece of aluminum foil and cut the absorbent insert along the longitudinal and lateral axis resulting in four individual specimens. On a calibrated balance, tare the weight of an appropriately sized bag. Place the specimen, and any material that fell onto the foil during cutting, into the tarred bag and heat seal the remaining open edge. Obtain and record the dry mass of the specimen to the nearest 0.001 grams. Repeat this procedure to obtain the dry mass of the remaining specimens. Three replicate absorbent inserts are prepared for testing.

Test Procedure

Obtain a pan large enough to hold several bags or specimens, and fill it with 0.9% saline solution to a level such that the bag or specimen may be completely submerged. Lay the bags or specimens to be tested onto the surface of the saline, and allow them to become wet for 1 minute before submerging. A weight placed onto the edge of the bag may be used to ensure that the sample remains submerged while allowing the solution to be freely absorbed by the specimen. After 30 minutes remove the bag or specimen from the saline solution, and immediately transfer it into the basket of a suitable centrifuge capable of subjecting the specimens to a 250 G centrifugal acceleration (such as a Clay Adams Dynac Centrifuge available from Block Scientific, Bohemia, N.Y.). Orient the specimens in the centrifuge such that the wearer-facing surface is facing outward, unless the wearer-facing surface is hydrophobic, in which case it should be oriented inward. Position the bags or specimens so that similarly weighted samples are opposite each other for proper balancing.

Centrifuge the bags or specimens for 3 minutes ± 10 seconds at 250 G. The bags or specimens are then removed and immediately weighed. Record the wet mass of the specimen and bag, blank bag, or specimen without a bag to the nearest 0.001 grams.

Calculate the average of the three wet blank bag masses after centrifugation ($m_b$); this value is disregarded if the specimen was tested without a bag. For each specimen (i=1, 2, and 3), calculate the centrifuge retention capacity ($w_i$), expressed as a mass fraction (g/g) using the following equation: $w_i = ((m_{wi} - m_b) - m_{si})/m_{si}$

Where $m_{si}$ is the mass, expressed in grams, of the dry specimen and $m_{wi}$ is the mass, expressed in grams, of the wet specimen and bag or specimen without a bag. For the absorbent inserts cut and tested as four separate specimens. Sum up the four individual masses of the dry specimens to get $m_{si}$, sum up the four individual masses of the wet specimens and bags to get $m_{wi}$, and sum up four of the average wet blank bag masses after centrifugation to get $m_b$. Use these values to calculate a total centrifuge retention capacity ($w_i$) value. Calculate and report the average centrifuge retention capacity for the three replicates to the nearest 0.001

Dynamic Shear Strength Method

The Dynamic Shear Strength Method is used to determine the force required to shear a fastening component from a receiving component and follows the general principles laid out in ASTM D5169-98. The resulting force is reported as the CD Shear Strength.

In this method, at least five specimens of like articles are analyzed. Fastening components and receiving components are excised from each article, optionally with the aid of cryogenic freeze spray if needed. If fusion bonds are present affixing the fastening or receiving components to one or more layers beneath, these bonds are left intact, and the included layers along with the hook are excised as a laminate and are included in subsequent testing.

In this method, the CD dimension of each of the fastening component and receiving component refer to the axes that were parallel to the lateral axis of the article prior to excision. (Similarly, the MD dimension of the fastening and receiving components are those that were parallel to the longitudinal dimension of the article prior to their excision.) The fastening component is placed on the receiving component in the approximate orientation in which the two components would be in contact in the article. If both the fastening component and receiving components are greater than 13 mm their respective CD dimensions, they are offset so as to overlap in the CD by 13 mm. Otherwise, the alignment in the CD dimension is chosen so as to maximize the CD dimension of overlap. The fastener is affixed to the receiving area, and the area of contact is rolled with a 5-kg roller as specified in ASTM D5169-98. The area of contact between the fastening component and receiving component is measured and recorded as the specimen contact area to the nearest square millimeter.

A tensile tester is configured with a 3-in gauge length, and the area of contact is centered between the clamps. If free portions of the fastening and/or receiving components are insufficiently long to be securely clamped, double-sided table is used to affix heavy paper extension leads of sufficient length to allow the specimen to be held securely in the clamps of the tensile tester. The specimen (with heavy paper leads affixed if necessary) is clamped in the tensile tester and the tensile tester's moving crosshead is moved (parallel to the CD dimension of both components) at 305 mm/min until the fastening component and receiving component are delaminated. The peak force in Newtons is recorded to the nearest 0.1 N as the unscaled specimen shear force. The unscaled specimen shear force is then normalized to correspond to a specimen contact area of 25.4 mm × 13 mm (or 330.2 mm$^2$) to arrive at the specimen shear strength (in Newtons to the nearest 0.1 N) for that specimen via the equation:

$$\text{specimen shear strength [N]} = \frac{330.2 \text{ mm}^2}{\text{specimen contact area [mm}^2]} \times \text{unscaled specimen shear force [N]}$$

At least five like specimens are analyzed in this way, and the arithmetic mean of all of the specimen shear strength values for all of the specimens is calculated and reported as the CD Shear Strength in units of Newtons to the nearest 0.1 N.

Peel Strength Method

The Peel Strength Method is used to determine the force required to peel a fastening component from a receiving component and follows the general principles laid out in ASTM D5170-98. The resulting force is reported as the CD Peel Strength.

In this method, at least five specimens of like articles are analyzed. Fastening components and receiving components are excised from each article, optionally with the aid of cryogenic freeze spray if needed. If fusion bonds are present affixing the fastening or receiving components to one or more layers beneath, these bonds are left intact, and the included layers along with the hook are excised as a laminate and are included in subsequent testing.

In this method, the CD dimension of each of the fastening component and receiving component refer to the axes that were parallel to the lateral axis of the article prior to excision. (Similarly, the MD dimension of the fastening and receiving components were those that were parallel to the longitudinal dimension of the article prior to their excision.) The fastening component is positioned relative to the receiving component in the orientation in which the two components would be in contact in the article and affixed to the receiving component and is rolled using the 5-kg roller specified in ASTM D5170-98. The overlap width is defined as the MD dimension of overlap between the hook component and the receiving component and is recorded to the nearest millimeter.

A tensile tester is configured with a 2 in gauge length. Double-sided table is used to affix heavy paper extension leads of sufficient length to allow the specimen to be held securely in the clamps of the tensile tester while performing a 180° "T-peel." The specimen (with heavy paper leads affixed) is clamped in the tensile tester and the tensile tester's moving crosshead is moved (parallel to the CD dimension of both components) at 305 mm/min until the fastening component and receiving component are separated. The peak force in Newtons is recorded to the nearest 0.1 N as the unscaled specimen peel strength. The unscaled specimen peel strength is then normalized to correspond to an overlap width of 25.4 mm to arrive at the specimen peel strength (in Newtons to the nearest 0.1 N) for that specimen via the equation:

$$\text{specimen peel strength [N]} = \frac{25.4 \text{ mm}}{\text{overlap width [mm]}} \times \text{unscaled specimen peel force [N]}$$

At least five like specimens are analyzed in this way, and the arithmetic mean of all of the specimen peel strength values for all of the specimens is calculated and reported as the CD Peel Strength in units of Newtons to the nearest 0.1 N.

Thickness Test Method

In general, the overall thickness of a fibrous material is measured as follows. Thickness measurements are performed using an Ono Sokki digital caliper (GS-503 Linear Gauge Sensor with DG-3610 Digital Gauge, Ono Sokki Co, Japan or equivalent) capable of measuring to 0.01 millimeters. The circular foot's diameter is 25.4 millimeters and the applied pressure is 0.689 kilopascals. At least five test samples of the same fibrous material are measured. Before testing, each of the test samples is placed on a flat surface and conditioned for 2 hours at $23 \pm 2\,°C$ and $50 \pm 2\,\%$ relative humidity. The measurements are performed under the same environmental conditions. For each test sample, three measurements are made at test sites corresponding to the longitudinal center of the test sample at 25%, 50% and 75% of the total lateral width of the test sample. For each measurement, the test sample is placed onto the caliper's anvil, with the body facing surface directed downward and with the test site centered under the foot. The foot is lowered at about 5 millimeters per second until the foot rests on the test sample. A reading from the caliper is taken after a residence time of 5 seconds and recorded to the nearest 0.01 millimeter. The foot is raised and the measurement is repeated in similar fashion at the other two test sites. The average of all measurements is reported to the nearest 0.01 millimeter. This test method can be used to measure the overall thickness of a fibrous material.

This test method can also be used to measure the overall thickness of a receiving component of a disposable wearable absorbent article. At least five test samples of the same receiving component are measured. Care should be taken to not to significantly stretch or distort the test sample when removing a receiving component from the article. If necessary, a cryogenic freeze spray (available as CytoFreeze, Control Company, TX or equivalent) can be used to facilitate removal of the receiving component.

Opacity Test Method

Opacity by contrast ratio measurements are made using a 0°/45° spectrophotometer suitable for making standard CIE L*a*b* color measurements (e.g. Hunterlab Labscan XE spectrophotometer, Hunter Associates Laboratory Inc., Reston VA or equivalent). The diameter of the instrument's measurement port should be chosen such that only the region of interest is included within the measurement port. Analyses are performed in a room controlled at about $23\,°C \pm 2\,C°$ and $50\,\% \pm 2\,\%$ relative humidity. Samples are conditioned at the same condition for 2 hours before testing.

Calibrate the instrument per the vendor instructions using the standard black and white tiles provided by the vendor. Set the spectrophotometer to use the CIE XYZ color space, with a D65 standard illumination and 10° observer. Using cryogenic spray and scissors carefully excise the specimen from the article for testing. Place the specimen flat against the instrument with the outward facing surface toward the spectrophotometer's measurement port and the region of interest within the port. Ensure that no tears, holes or apertures are within the measurement port. Place the white standard tile onto the opposing surface of the specimen such that it completely covers the measurement port. Take a reading for XYZ and record to 0.01 units. Without moving the specimen, remove the white plate and replace it with the black standard plate. Take a second reading for XYZ and record to 0.01 units. Repeat this procedure at a corresponding site for a total of ten (10) replicate specimens.

Opacity is calculated by dividing the Y value measured using the black tile as backing, divided by the Y value measured using the white tile as backing. Record the opacity value to the nearest 0.001. Calculate opacity for the 10 replicates for the sample and report the average opacity to the nearest 0.001.

The difference between an opacity of a first sample and a second sample is calculated using the following equation:

$$\%Difference = \frac{average\ opacity\ for\ sample\ 1 - average\ opacity\ of\ sample\ 2}{average\ opacity\ of\ sample\ 2} * 100\%$$

wherein sample 2 is the sample having the lower of the two average opacities.

The citation of any document is not an admission that it is prior art with respect to any invention disclosed or claimed herein or that it alone, or in any combination with any other reference or references, teaches, suggests or discloses any such invention.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this inventior defined by the claims.

## Claims

1. An absorbent article (10) comprising:

   an absorbent insert (30) comprising:
   a topsheet, a backsheet, and an absorbent core disposed between the topsheet and backsheet;
   an outer cover (20);
   a fastening system (100) for securing the absorbent insert (30) to the outer cover (20), the fastening system comprising a fastening component (110) and a receiving component (112) operatively engageable with the fastening component, wherein:

   the receiving component is disposed on a surface of one of the absorbent insert or the outer cover (30, 20) and the fastening component is disposed on another of the absorbent insert or outer cover (20, 30), the surface comprising a surface area;
   the receiving component comprises an area that is at least 50% of the surface area; and
   wherein the fastening component comprises a first macro pattern (120) of fastening elements (114) and a second macro pattern (120b) of fastening elements, wherein:

   the first and second macro patterns differ by peel strength, as determined by the method set out in the description, surface area, directionality of fastening elements, whether fastening elements are discrete or integral or some combination, fastening element constituent materials, the number of fastening elements, the number and/or types of layers from which integral fastening elements are formed, and combinations thereof; and/or;
   the first and second macro patterns each comprises a plurality of arrays of fastening elements (122a, 122b, 122c), and the first and second macro patterns differ by average size of fastening elements within an array, average spacing of fastening elements within an array, aggregate shapes of arrays, orientation of arrays, spacing of arrays from one another, and combinations thereof.

2. The absorbent article of claim 1, wherein the fastening component comprises integrally formed hooks (116).

3. The absorbent article of any of the preceding claims, wherein the receiving component comprises integrally formed loops (125).

4. The absorbent article of any of the preceding claims, wherein the absorbent article comprises at least about 10% bio-based content.

5. The absorbent article any of the preceding claims, wherein the outer cover is reusable and the absorbent insert is disposable.

6. The absorbent article of any of the preceding claims, wherein the fastening component comprises a micro pattern having a maximum width, Wp, that is at least about 50% of the width of the absorbent insert.

7. The absorbent article of any of the preceding claims, wherein the fastening component comprises fastening elements at least partially overlapping the longitudinal axis.

8. The absorbent article of any of the preceding claims, wherein the fastening component comprises fastening elements at least partially overlapping the lateral axis.

9. The absorbent article of any of the preceding claims, wherein the absorbent insert has an absorbent capacity of at least 120 g, as determined by the Centrifuge Retention Capacity Test method set out in the description.

10. The absorbent article of any of the preceding claims, wherein the outer cover comprises the receiving component and the absorbent insert comprises the fastening component.

11. The absorbent article of any of the preceding claims, wherein the outer cover comprises the fastening component and the absorbent insert comprises the receiving component.

12. The absorbent article of any of the preceding claims, wherein the fastening component at least partially overlaps the longitudinal axis of absorbent insert when the article is assembled.

13. The absorbent article of any of the preceding claims, wherein the receiving component comprises a plurality of fibers, and a majority of the fibers have a cross-sectional dimension of 5 to 150 micrometers.

14. The absorbent article of any of the preceding claims, wherein the fastening component comprises a maximum length that is about 30% or less of a maximum length of the receiving component.

**Patentansprüche**

1. Absorptionsartikel (10), umfassend:
   einen Absorptionseinsatz (30), umfassend:

   eine Oberschicht, eine Unterschicht und einen Absorptionskern, der zwischen der Oberschicht und der Unterschicht eingerichtet ist;
   einen Außenmantel (20);
   ein Befestigungssystem (100) zum Befestigen des Absorptionseinsatzes (30) an dem Außenmantel (20), das Befestigungssystem umfassend einen Befestigungsbestandteil (110) und einen Aufnahmebestandteil (112), der mit dem Befestigungsbestandteil wirksam in Eingriff bringbar ist, wobei:

   der Aufnahmebestandteil auf einer Oberfläche des Absorptionseinsatzes oder des Außenmantels (30, 20) eingerichtet ist und der Befestigungsbestandteil auf einer anderen des Absorptionseinsatzes oder des Außenmantels (20, 30) eingerichtet ist, die Oberfläche umfassend einen Oberflächenbereich;
   wobei der Aufnahmebestandteil einen Bereich, der wenigstens 50 % der Oberfläche beträgt, umfasst, und
   wobei der Befestigungsbestandteil ein erstes Makromuster (120) aus Befestigungselementen (114) und ein zweites Makromuster (120b) aus Befestigungselementen umfasst, wobei:

   sich das erste und das zweite Makromuster durch Schälfestigkeit, wie durch das in der Beschreibung dargelegte Verfahren bestimmt, Oberflächenbereich, Richtungsabhängigkeit von Befestigungselementen, darin ob Befestigungselemente separat oder integral oder eine Kombination sind, Befestigungselement-Bestandteilsmaterialien, die Anzahl von Befestigungselementen, die Anzahl und/oder Arten von Schichten, aus denen integrale Befestigungselemente geformt sind, und Kombinationen davon, unterscheiden; und/oder;
   das erste und das zweite Makromuster jeweils eine Vielzahl von Anordnungen von Befestigungselementen (122a, 122b, 122c) umfasst, und sich das erste und das zweite Makromuster durch eine durchschnittliche Größe von Befestigungselementen innerhalb einer Anordnung, eine durchschnittliche Beabstandung von Befestigungselementen innerhalb einer Anordnung, aggregierte Formen von Anordnungen, eine Ausrichtung von Anordnungen, eine Beabstandung von Ausrichtungen voneinander und Kombinationen davon, unterscheiden.

2.  Absorptionsartikel nach Anspruch 1, wobei der Befestigungsbestandteil integral geformte Haken (116) umfasst.

3.  Absorptionsartikel nach einem der vorstehenden Ansprüche, wobei der Aufnahmebestandteil integral geformte Schlaufen (125) umfasst.

4.  Absorptionsartikel nach einem der vorstehenden Ansprüche, wobei der Absorptionsartikel wenigstens etwa zu 10 % einen biobasierten Gehalt umfasst.

5.  Absorptionsartikel nach einem der vorstehenden Ansprüche, wobei der Außenmantel wiederverwendbar ist und der Absorptionseinsatz ein Einweg-Absorptionseinsatz ist.

6.  Absorptionsartikel nach einem der vorstehenden Ansprüche, wobei der Befestigungsbestandteil ein Mikromuster, das eine maximalen Breite, Wp, aufweist, die wenigstens etwa 50 % der Breite des Absorptionseinsatzes beträgt, umfasst.

7.  Absorptionsartikel nach einem der vorstehenden Ansprüche, wobei der Befestigungsbestandteil Befestigungselemente, die die Längsachse wenigstens teilweise überlappen, umfasst.

8.  Absorptionsartikel nach einem der vorstehenden Ansprüche, wobei der Befestigungsbestandteil Befestigungselemente, die die Querachse wenigstens teilweise überlappen, umfasst.

9.  Absorptionsartikel nach einem der vorstehenden Ansprüche, wobei der Absorptionseinsatz ein Absorptionsvermögen von wenigstens 120 g aufweist, wie durch das in der Beschreibung dargelegte Prüfverfahren für die Zentrifugenretentionsvermögen bestimmt.

10. Absorptionsartikel nach einem der vorstehenden Ansprüche, wobei der Außenmantel den Aufnahmebestandteil umfasst und der Absorptionseinsatz den Befestigungsbestandteil umfasst.

11. Absorptionsartikel nach einem der vorstehenden Ansprüche, wobei der Außenmantel den Befestigungsbestandteil umfasst und der Absorptionseinsatz den Aufnahmebestandteil umfasst.

12. Absorptionsartikel nach einem der vorstehenden Ansprüche, wobei der Befestigungsbestandteil die Längsachse des Absorptionseinsatzes wenigstens teilweise überlappt, wenn der Artikel zusammengebaut ist.

13. Absorptionsartikel nach einem der vorstehenden Ansprüche, wobei der Aufnahmebestandteil eine Vielzahl von Fasern umfasst und eine Mehrheit der Fasern eine Querschnittsabmessung von 5 bis 150 Mikrometer aufweist.

14. Absorptionsartikel nach einem der vorstehenden Ansprüche, wobei der Befestigungsbestandteil eine maximale Länge, die etwa 30 % oder weniger einer maximalen Länge des Aufnahmebestandteils beträgt, umfasst.

**Revendications**

1.  Article absorbant (10) comprenant :
    un article absorbant (30) comprenant :

    une feuille de dessus, une feuille de fond et une âme absorbante disposée entre la feuille de dessus et la feuille de fond ;
    une couverture externe (20) ;
    un système de fixation (100) pour fixer l'insert absorbant (30) au couvercle extérieur (20), le système de fixation comprenant un composant de fixation (110) et un composant de réception (112) pouvant être engagé de manière opérationnelle avec le composant de fixation, dans lequel :

l'élément de réception est disposé sur une surface de l'insert absorbant ou de la couverture extérieure (30, 20) et l'élément de fixation est disposé sur une autre surface de l'insert absorbant ou de la couverture extérieure (20, 30), la surface comprenant une zone superficielle ;

l'élément récepteur comprend une zone qui représente au moins 50 % de la zone superficielle.
et

dans lequel le composant de fixation comprend un premier macro-modèle (120) d'éléments de fixation (114) et un second macro-modèle (120b) d'éléments de fixation, dans lequel :

le premier et le second macro-modèles diffèrent par la résistance au pelage, telle que déterminée par le procédé décrit dans la description, la zone superficielle, la directionnalité des éléments de fixation, le fait que les éléments de fixation soient discrets ou intégraux ou une combinaison des deux, les matériaux constitutifs des éléments de fixation, le nombre d'éléments de fixation, le nombre et/ou les types de couches à partir desquelles les éléments de fixation intégraux sont formés, et les combinaisons de ces éléments ; et/ou ;

les premier et second macro-modèles comprennent chacun une pluralité de plusieurs réseaux d'éléments de fixation (122a, 122b, 122c), et les premier et second macro-modèles diffèrent par la taille moyenne des éléments de fixation dans un réseau, l'espacement moyen des éléments de fixation dans un réseau, les formes globales des réseaux, l'orientation des réseaux, l'espacement des réseaux les uns par rapport aux autres, et des combinaisons de ces éléments.

2. Article absorbant selon la revendication 1, dans lequel le composant de fixation comprend des crochets intégralement formés (116).

3. Article absorbant selon l'une quelconque des revendications précédentes, dans lequel le composant de réception comprend des boucles intégralement formées (125).

4. Article selon l'une quelconque des revendications précédentes, dans lequel l'article absorbant comprend au moins environ 10 % de contenu organique.

5. Article absorbant selon l'une quelconque des revendications précédentes, dans lequel le couvercle extérieur est réutilisable et l'insert absorbant est jetable.

6. Article absorbant selon l'une quelconque des revendications précédentes, dans lequel le composant de fixation comprend un micro-modèle ayant une largeur maximale, Wp, qui est au moins d'environ 50 % de la largeur de l'insert absorbant.

7. Article absorbant selon l'une quelconque des revendications précédentes, dans lequel le composant de fixation comprend des éléments de fixation chevauchant au moins partiellement l'axe longitudinal.

8. Article absorbant selon l'une quelconque des revendications précédentes, dans lequel le composant de fixation comprend des éléments de fixation chevauchant au moins partiellement l'axe latéral.

9. Article absorbant selon l'une quelconque des revendications précédentes, dans lequel l'insert absorbant a une capacité d'absorption d'au moins 120 g, telle que déterminée par le procédé de test de capacité de rétention en centrifugeuse exposé dans la description.

10. Article absorbant selon l'une quelconque des revendications précédentes, dans lequel le couvercle extérieur comprend le composant de réception et l'insert absorbant comprend le composant de fixation.

11. Article absorbant selon l'une quelconque des revendications précédentes, dans lequel le couvercle extérieur comprend l'élément de fixation et l'insert absorbant comprend l'élément de réception.

12. Article absorbant selon l'une quelconque des revendications précédentes, dans lequel l'élément de fixation chevauche au moins partiellement l'axe longitudinal de l'insert absorbant lorsque l'article est assemblé.

13. Article absorbant selon l'une quelconque des revendications précédentes, dans lequel le composant récepteur comprend une pluralité de fibres, et une majorité des fibres ont une dimension de section transversale de 5 à 150 micromètres.

14. Article absorbant selon l'une quelconque des revendications précédentes, dans lequel l'élément de fixation comprend une longueur maximale qui est d'environ 30 % ou moins de la longueur maximale de l'élément de réception.

Fig. 1

Fig. 2A

Fig. 2B

Fig. 2C

EP 4 103 120 B1

Fig. 3

Fig. 4

Fig. 5

Fig. 6A

Fig. 6B

Fig. 7A

Fig. 7B

Fig. 7C

1HD

222

Fig. 8A

220

223

Fig. 8B

221

223

Fig. 8C

222

Fig. 9A

220

223

221

223

Fig. 9B  Fig. 9C

2HD

222

Fig. 10A

220

224

224

223

Fig. 10B

221

223

Fig. 10C

Fig. 11

Fig. 12A

Fig. 12B

Fig. 12C

Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2012022485 A1
- US 2013006209 A
- US 6120487 A
- US 8998870 B
- US 9089456 B
- US 8435223 B
- US 9011402 B
- US 8808263 B
- US 8759605 B
- US 8932273 B
- US 9078789 B
- US 687493
- US 12687412
- US 12687528
- US 12687425, Roe
- US 7223818 B
- US 7211531 B
- US 7060149 B
- US 6964720 B
- US 6905987 B
- US 6890872 B
- US 6884494 B
- US 6878647 B
- US 5518801 A
- US 20080319407 A
- US 20080045917 A
- US 20070293111 A
- US 20070287983 A
- US 20070287348 A
- US 20070249254 A
- US 20070203301 A
- US 20050164587 A
- US 533472
- US 15074675 B
- US 62855001 B
- US 8546641 B
- US 5628097 A, Benson
- US 20160136014 A, Arora
- US 4573986 A
- US 3911173 A
- US 4785996 A
- US 4842666 A
- US 4610678 A
- US 4673402 A
- US 4834735 A
- US 4888231 A
- US 5137537 A
- US 5147345 A
- US 5342338 A
- US 5260345 A
- US 5387207 A
- US 5397316 A
- US 491642
- US 15232901 B
- WO 200059430 A
- WO 9510996 A
- US 5700254 A
- WO 02067809 A
- US 62134622
- US 14077708 B
- US 8939957 B
- US 3860003 A
- US 7435243 B
- US 8062279 B
- US 684230
- US 16545425 B
- US 3848594 A
- US 4662875 A
- US 4846815 A
- US 4894060 A
- US 4946527 A
- US 5151092 A
- US 5221274 A
- US 6432098 B
- US 8784722 B
- US 545425
- US 20070219521 A1
- US 20110139658 A1
- US 20110139657 A1
- US 20110152812 A1
- US 20110139659 A1